(19) Europäisches Patentamt
European Patent Office
Office européen des brevets

(11) EP 0 647 226 B1

# (12) EUROPÄISCHE PATENTSCHRIFT

(45) Veröffentlichungstag und Bekanntmachung des Hinweises auf die Patenterteilung:
**03.09.1997 Patentblatt 1997/36**

(51) Int Cl.$^6$: **C07D 471/14**, A61K 31/50
// (C07D471/14, 237:00, 221:00, 209:00)

(21) Anmeldenummer: **93913008.4**

(22) Anmeldetag: **18.06.1993**

(86) Internationale Anmeldenummer:
**PCT/EP93/01553**

(87) Internationale Veröffentlichungsnummer:
**WO 94/00451 (06.01.1994 Gazette 1994/02)**

## (54) 9-AMINO-PYRIDAZINO 4',5' : 3,4]PYRROLO- 2,1-a]ISOCHINOLINE UND DEREN VERWENDUNG FÜR DIE HERSTELLUNG VON PHARMAZEUTISCHEN ZUBEREITUNGEN

9-AMINOPYRIDAZINO 4',5' : 3,4]pyrrolo 2,1-a]ISOQUINOLINES AND THEIR USE IN THE PREPARATION OF PHARMACEUTICAL COMPOSITIONS

9-AMINO-PYRIDAZINO 4',5' : 3,4]PYRROLO 2,1-a]ISOQUINOLEINES ET LEUR UTILISATION DANS LA FABRICATION DE PREPARATIONS PHARMACEUTIQUES

(84) Benannte Vertragsstaaten:
**AT BE CH DE DK ES FR GB GR IE IT LI LU MC NL PT SE**

(30) Priorität: **22.06.1992 DE 4220384**
**22.06.1992 DE 4220361**
**22.06.1992 DE 4220380**

(43) Veröffentlichungstag der Anmeldung:
**12.04.1995 Patentblatt 1995/15**

(73) Patentinhaber:
- **BOEHRINGER INGELHEIM KG**
  **55216 Ingelheim (DE)**
  Benannte Vertragsstaaten:
  **BE CH DE DK ES FR GR IT LI LU MC NL PT SE AT**
- **BOEHRINGER INGELHEIM INTERNATIONAL GmbH**
  **55216 Ingelheim am Rhein (DE)**
  Benannte Vertragsstaaten:
  **GB IE**

(72) Erfinder:
- **ARNDTS, Dietrich**
  **D-6531 Appenheim (DE)**
- **LÖSEL, Walter**
  **D-6535 Gau-Algesheim (DE)**
- **ROOS, Otto**
  **D-6501 Schwabenheim (DE)**

(56) Entgegenhaltungen:
**EP-A- 0 150 474**
**EP-A- 0 190 563**
**EP-A- 0 252 299**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 647 226 B1

## Beschreibung

Die Erfindung betrifft die Verwendung von 9-Amino-pyridazino[4',5' : 3,4] pyrrolo [2,1-a]-isochinolinen der Formel

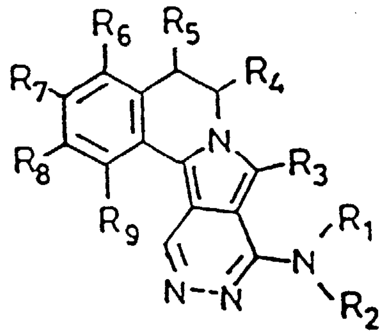

(I)

sowie deren physiologisch verträglichen Salzen mit Säuren und Komplexbildnern zur Herstellung von Mitteln zur Behandlung chronisch inflammatorischer Prozesse, der Colitis Ulcerosa und des Morbus Crohn und von Mitteln mit antiproliferativer Wirkung.

Wie weiter unten ausgeführt wird, ist ein Teil dieser Verbindungen aus DE 35 00 941, DE 35 25 048, EP 190 563 und EP 252 299 bekannt, ein Teil dieser Verbindungen ist jedoch neu. Die vorliegende Erfindung betrifft daher auch diese neuen Verbindungen, diese Verbindungen enthaltende pharmazeutische Zubereitungen, insbesondere deren Verwendung als hirnprotektive Mittel, insbesondere bei der Behandlung von Patienten, die einen Schlaganfall erlitten haben, oder gefährdet sind, einen Schlaganfall zu erleiden.

In Formel I bedeuten:

$R_1$ und $R_2$, die gleich oder verschieden sein können, Wasserstoff; $C_3$-$C_7$ Cycloalkyl; $C_2$-$C_5$ Alkenyl; Phenyl (wobei der Phenylring gegebenenfalls ein- oder zweifach durch Halogen oder Methoxy substituiert ist); Propargyl; einen geradkettigen oder verzweigten, gesättigten oder ungesättigten Alkylrest mit 1 - 5 Kohlenstoffatomen, der substituiert sein kann durch Hydroxy, Alkoxy mit 1 - 4 Kohlenstoffatomen, Halogen, $NH_2$, NH-Alkyl mit 1 - 2 Kohlenstoffatomen, N,N-Di($C_1$-$C_2$)alkylamino, NH-Acyl mit 2 - 4 Kohlenstoffatomen, Cycloalkyl mit 3 - 7 Kohlenstoffatomen, 1 oder 2 Phenylgruppen (wobei der/die Phenylring(e) seinerseits (ihrerseits) ein- oder zweifach substituiert sein kann (können) durch Halogen, $CF_3$, Alkyl mit 1 - 4 Kohlenstoffatomen, Alkoxy mit 1 - 2 Kohlenstoffatomen, NH-Alkyl mit 1 - 2 Kohlenstoffatomen, N,N-Dialkyl mit 1 - 2 Kohlenstoffatomen, $NH_2$, N-Acyl mit 2 - 3 Kohlenstoffatomen, Alkylsulfonylamino oder Benzyloxy), Furyl, Thienyl, einen stickstoffhaltigen heterocyclischen 5- oder 6-Ring der gegebenenfalls als weiteres Heteroatom ein Sauerstoff- oder Schwefelatom enthalten kann (wobei der Ring gegebenenfalls durch Alkyl mit 1 - 4 Kohlenstoffatomen substituiert ist); oder

$R_1$ und $R_2$ zusammen mit dem Stickstoffatom einen 3- bis 7-gliedrigen Ring, der gegebenenfalls als weiteres Heteroatom ein Sauerstoff- oder ein Stickstoffatom enthalten kann, wobei dieser Ring gegebenenfalls durch Phenyl-($C_0$-$C_4$)-Alkyl substituiert ist (wobei der Phenylring seinerseits ein- oder zweifach durch Halogen, $CF_3$, ($C_1$-$C_4$) Alkoxy, ($C_1$-$C_4$)Alkyl oder CN substituiert sein kann, wobei die Substituenten gleich oder verschieden voneinander sein können); oder

$R_2$, falls $R_1$ Wasserstoff bedeutet, auch $-NH_2$; Di($C_1$-$C_2$) Alkylamino; Acetonylamino; -NH($C_2$-$C_3$)Acyl; einen Alkylsulfonyl- oder Alkoxycarbonylrest mit je 1 - 3 Kohlenstoffatomen in der Alkylkette; die Isopropylidenaminogruppe

( 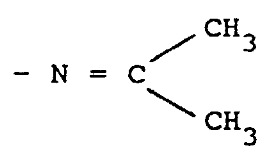 )

oder einen heterocyclischen, ein Stickstoffatom und gegebenenfalls als weiteres Heteroatom ein Sauerstoff-, Stickstoff- oder Schwefelatom enthaltenden 5- oder 6-Ring bedeuten kann;

$R_3$, $R_4$ und $R_5$, die gleich oder verschieden sein können, Wasserstoff oder einen Alkylrest mit 1 - 4 Kohlenstoff-

atomen;

$R_7$ und $R_8$, die gleich oder verschieden sein können, Hydroxy; Alkoxy mit 1 - 4 Kohlenstoffatomen; oder Alkylthio mit 1 - 4 Kohlenstoffatomen und

$R_6$ und $R_9$, die gleich oder verschieden sein können, Wasserstoff; Hydroxy; Alkoxy mit 1 - 4 Kohlenstoffatomen; Alkylthio mit 1 - 4 Kohlenstoffatomen; oder den Rest

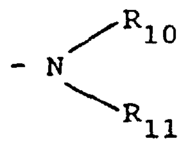 ,

in dem

$R_{10}$ Wasserstoff; oder Alkyl mit 1 - 4 Kohlenstoffatomen ist und
$R_{11}$ Wasserstoff; oder Alkyl mit 1 - 4 Kohlenstoffatomen, wobei der Alkylrest gegebenenfalls durch Hydroxy, Methoxy oder Furfuryl substituiert sein kann;

oder 2 benachbarte Substituenten der Substituenten $R_6$, $R_7$, $R_8$ und $R_9$ zusammen die Gruppe -O-$(CH_2)_1$ oder 2-O- bilden und die jeweils übrigen 2 Substituenten wie oben definiert sind.

Gegenstand der Erfindung ist auch die Verwendung deren physiologisch verträgliche Salze mit Säuren, Basen oder Komplexbildnern zur Herstellung von Mitteln zur Behandlung chronisch inflammatorischer Prozesse, der Colitis Ulcerosa und des Morbus Crohn, und von Mitteln mit antiproliferativer Wirkung.

Gegenstand der Erfindung sind wie oben erwähnt, auch die neuen Verbindungen der allgemeinen Formel I worin $R_3$, $R_4$, $R_5$, $R_6$ und $R_9$ Wasserstoff sind, $R_7$ und $R_8$ Alkoxy mit 1-4 Kohlenstoffatomen sind oder $R_7$ und $R_8$ zusammen $-OCH_2O-$ oder $-OCH_2CH_2O-$ sind, und die Gruppe $NR_1R_2$

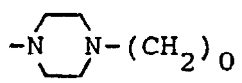 oder 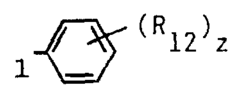

ist, worin z null 1 oder 2 ist und $R_{12}$ CN, $CF_3$, Halogen, $(C_1$-$C_4)$Alkyl oder $(C_1$-$C_4)$Alkoxy ist, oder deren physiologisch verträgliche Salze mit Säuren oder Komplexbindnern ausgenommen die Verbindung

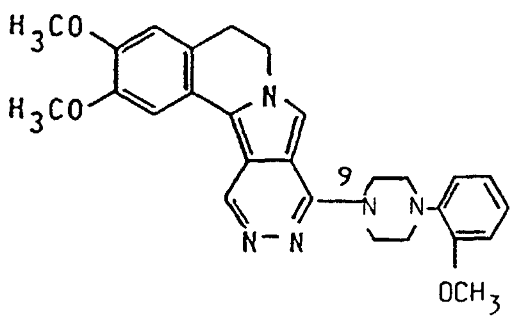

Bevorzugte Bereiche der erfindungsgemäßen Verwendung:
Verwendung einer Verbindung der Formel (I) nach obiger Definition die die alten und neuen Verbindungen umfaßt in der

$R_1$ und $R_2$, die gleich oder verschieden sein können, Wasserstoff; $C_3$-$C_7$ Cycloalkyl; $C_2$-$C_5$ Alkenyl; Phenyl (wobei der Phenylring gegebenenfalls ein- oder zweifach durch Halogen oder Methoxy substituiert ist); Propargyl; einen geradkettigen oder verzweigten, gesättigten oder ungesättigten Alkylrest mit 1 - 5 Kohlenstoffatomen, der substituiert sein kann durch Hydroxy, Alkoxy mit 1 - 4 Kohlenstoffatomen, Halogen, $NH_2$, NH-Alkyl mit 1 - 2 Kohlenstoffatomen, N,N-Di($C_1$-$C_2$)alkylamino, NH-Acyl mit 2 - 4 Kohlenstoffatomen, Cycloalkyl mit 3 - 7 Kohlenstoffatomen, Phenyl (wobei der Phenylring seinerseits ein- oder zweifach substituiert sein kann durch Halogen, Alkyl mit 1 - 2 Kohlenstoffatomen, Alkoxy mit 1 - 2 Kohlenstoffatomen, NH-Alkyl mit 1 - 2 Kohlenstoffatomen, N,N-Dialkyl mit 1 - 2 Kohlenstoffatomen, $NH_2$, N-Acyl mit 2 - 3 Kohlenstoffatomen oder Alkylsulfonylamino), Furyl, Thienyl, einen stickstoffhaltigen heterocyclischen 5- oder 6-Ring der gegebenenfalls als weiteres Heteroatom ein Sauerstoff- oder Schwefelatom enthalten kann (wobei der Ring gegebenenfalls durch Alkyl mit 1 - 4 Kohlenstoffatomen substituiert ist) bedeuten; oder

$R_1$ und $R_2$ zusammen mit dem Stickstoffatom einen 3- bis 7-gliedrigen Ring, der gegebenenfalls als weiteres Heteroatom ein Sauerstoff- oder ein Stickstoffatom enthalten kann, wobei dieser Ring gegebenenfalls durch Phenyl-($C_0$-$C_4$)-Alkyl substituiert ist (wobei der Phenylring seinerseits ein- oder zweifach durch Halogen oder Methoxy substituiert ist) bedeuten; oder

$R_2$, falls $R_1$ Wasserstoff bedeutet, auch $-NH_2$; Di($C_1$-$C_2$) Alkylamino; Acetonylamino; -NH($C_2$-$C_3$)Acyl; einen Alkylsulfonyl- oder Alkoxycarbonylrest mit je 1 - 3 Kohlenstoffatomen in der Alkylkette; die Isopropylidenaminogruppe

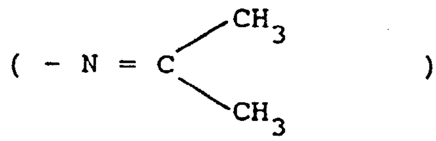

oder einen heterocyclischen, ein Stickstoffatom und gegebenenfalls als weiteres Heteroatom ein Sauerstoff-, Stickstoff- oder Schwefelatom enthaltenden 5- oder 6-Ring bedeuten kann;

$R_3$, $R_4$ und $R_5$, die gleich oder verschieden sein können, Wasserstoff oder einen Alkylrest mit 1 - 4 Kohlenstoffatomen bedeuten;

$R_7$ und $R_8$, die gleich oder verschieden sein können, Hydroxy; Alkoxy mit 1 - 4 Kohlenstoffatomen; oder Alkylthio mit 1 - 4 Kohlenstoffatomen bedeuten und

$R_6$ und $R_9$, die gleich oder verschieden sein können, Wasserstoff; Hydroxy; Alkoxy mit 1 - 4 Kohlenstoffatomen; Alkylthio mit 1 - 4 Kohlenstoffatomen; oder den Rest

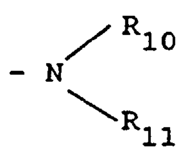

bedeuten, in dem

$R_{10}$ Wasserstoff; oder Alkyl mit 1 - 4 Kohlenstoffatomen ist und
$R_{11}$ Wasserstoff; oder Alkyl mit 1 - 4 Kohlenstoffatomen, wobei der Alkylrest gegebenenfalls durch Hydroxy, Methoxy oder Furfuryl substituiert sein kann;

sowie deren physiologisch verträglichen Salzen mit Säuren oder Komplexbildnern zur Herstellung von Mitteln zur Behandlung chronisch inflammatorischer Prozesse, der Colitis Ulcerosa und des Morbus Crohn, und von Mitteln mit antiproliferativer Wirkung.

-) Verwendung einer Verbindung I, worin $-NR_1R_2$ für

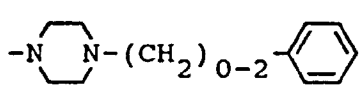

steht,
worin die Phenylgruppe durch eine oder zwei Methoxygruppen substituiert sein kann.

-) Verwendung einer Verbindung (I), worin $-NR_1R_2$ die Gruppe

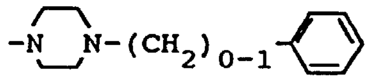

ist, worin die Phenylgruppe wie oben definiert substituiert sein kann.

-) Verwendung einer Verbindung (I), worin $-NR_1R_2$ die Gruppe

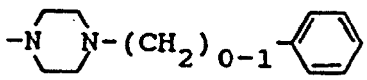

ist, worin der Phenylring ein- oder zweifach durch Fluor, Chlor, $CF_3$, Methoxy, Methyl, Ethyl oder CN substiutiert ist.

-) Verwendung einer Verbindung (I) worin $R_1$ Wasserstoff ist und $R_2$ ein geradkettiger oder verzweigter Alkylrest mit 1 bis 4 Kohlenstoffatomen ist, der durch Cycloalkyl mit 3 bis 7 Kohlenstoffatomen, Thienyl oder 1 oder 2 unsubstituierte Phenylgruppen oder durch eine substituierte Phenylgruppe, deren Substituent(en) wie oben definiert sind, substituiert ist.

-) Verwendung einer Verbindung (I), worin $R_1$ Wasserstoff ist und $R_2$ $(C_1$-$C_4)$Alkylcyclohexyl, vorzugsweise $-CH_2$-$C_6H_{11}$ ist.

-) Verwendung einer Verbindung (I), worin $R_1$ Wasserstoff ist und $R_2$ $(C_1$-$C_4)$Alkylphenyl, worin die Phenylgruppe unsubstituiert ist oder ein- oder zweifach substituiert ist durch F, Cl, $CF_3$, Methyl, Ethyl, Methoxy oder Ethoxy.

-) Verwendung einer Verbindung (I), worin $NR_1R_2$ eine der folgenden Gruppen ist:

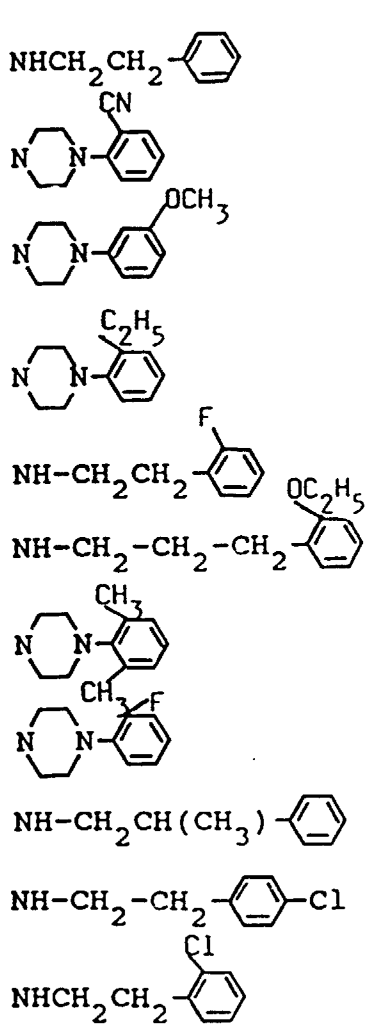

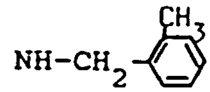

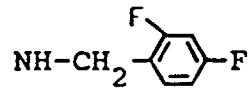

$NH-CH_2-CH(C_6H_5)_2$

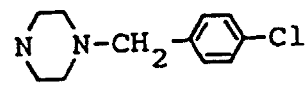

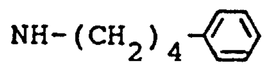

$NH-CH_2-C_6H_5$

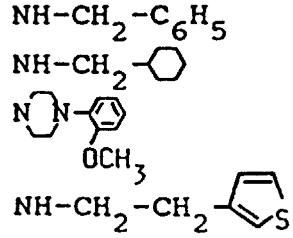

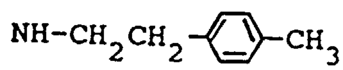

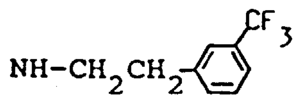

$NH-CH_2-CH_2-CH(C_6H_5)_2$

-) Verwendung einer Verbindung (I), worin $NR_1R_2$ eine der folgenden Gruppen ist:

$NH-CH_2-C_6H_5$

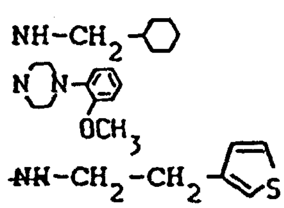

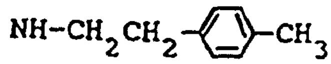

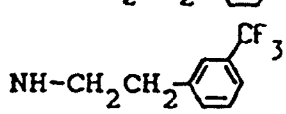

$NH-CH_2-CH_2-CH(C_6H_5)_2$

-) Verwendung einer Verbindung (I), worin $R_3$, $R_4$, $R_5$, $R_6$ und $R_9$ Wasserstoff sind und $R_7$ und $R_8$ Alkoxy mit 1-4 Kohlenstoffatomen oder $R_7$ und $R_8$ zusammen $-OCH_2O-$ oder $-OCH_2CH_2O-$ sind.

-) Verwendung einer Verbindung (I), worin $R_7$ und $R_8$ Methoxy sind.

Bevorzugt ist ferner die Verwendung solcher Verbindungen der allgemeinen Formel I, in denen $R_1$ und $R_2$, die gleich oder verschieden sein können für Wasserstoff; eine geradkettige oder verzweigte Alkylgruppe mit 1 - 5 Kohlenstoffatomen oder $R_1$ für Wasserstoff und $R_2$ für Amino; Methylamino; Dimethylamino; Isopropylidenamino; Dimethylamino-$C_1$-$C_4$-alkyl; Methoxy-$C_1$-$C_4$-alkyl; Cyclopropyl; Cyclopentyl; Cyclohexyl; Cyclohexylmethyl; Phenyl; Phenylethyl, wobei der Phenylring gegebenenfalls ein- oder zweifach durch Methoxy oder Halogen substituiert ist; Pyrazolyl; Propargyl; [1-Methylpyrrolidin-2-yl]-ethyl; (Piperidin-1-yl)ethyl; Allyl; 4-Benzyl-piperazin-1-yl; (Furan-2-yl)methyl; (Pyrrolidin-1-yl)-ethyl; 2-Hydroxyethyl; (Pyridin-4-yl)-ethyl; Benzyl; (Thien-3-yl) ethyl; oder $R_1$ und $R_2$ zusammen mit dem Stickstoffatom an welches sie gebunden sind für Pyrrolidin; Morpholin; Piperazin welches gewünschtenfalls durch Phenethyl oder Methoxyphenyl substituiert ist; stehen
und

$R_7$ und $R_8$ unabhängig voneinander für Methoxy; Hydroxy; oder Methylthio

und
$R_3$, $R_4$, $R_5$, $R_6$ und $R_9$ für Wasserstoff stehen.

Die Verbindungen der Formel I sind Basen und können auf übliche Weise mit anorganischen oder organischen Säuren sowie Salz- und Komplexbildnern in beliebige physiologisch unbedenkliche Addukte (Salze) überführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, oxalsäure, Malonsäure, Bernsteinsäure, Maleinsäure, Fumarsäure, Milchsäure, Weinsäure, Zitronensäure, Apfelsäure, Benzoesäure, p-Hydroxybenzoesäure, Phthalsäure, Zimtsäure, Salicylsäure, Ascorbinsäure, Methansulfonsäure und dergleichen.

Bevorzugt werden Verbindungen der allgemeinen Formel I verwendet, worin $R_3$, $R_4$, $R_5$, $R_6$ und $R_9$ Wasserstoff sind und $R_7$ und $R_8$ Methoxy sind, und/oder

$R_1$ Wasserstoff ist und $R_2$ eine Gruppe $-(CH_2)_{0-3}-A$, worin A
Cyclopentyl, Cyclohexyl, Phenyl, Mono- oder Dimethoxyphenyl

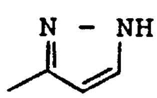 , 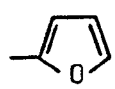 oder 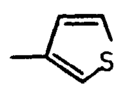

ist,

oder $R_2$ verzweigtes oder unverzweigtes Alkyl mit 4 oder 5 Kohlenstoffatomen ist; insbesondere Verbindungen worin die Gruppe

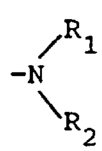

für

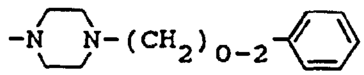

steht, worin die Phenylgruppe durch eine oder zwei Methoxygruppen substituiert sein kann; oder $R_1$ Wasserstoff ist und $R_2$ eine der folgenden Gruppen ist

Cyclopentyl
Cyclohexyl
Phenyl

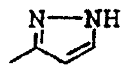

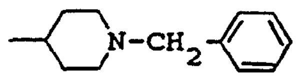

$-CH_2-C_6H_5$

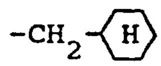

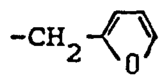

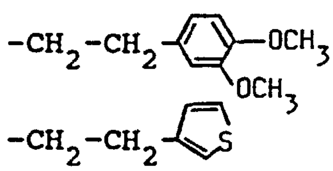

$-(CH_2)_3CH_3$

$-(CH_2)_4CH_3$

$-CH_2-CH(CH_3)_2$

$-(CH_2)_2-CH(CH_3)_2$

$-(CH_2)_3-OCH_3$

oder die Gruppe

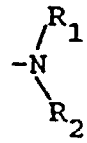

für

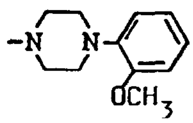

oder

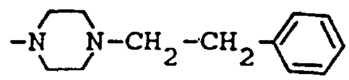

steht, insbesondere worin $R_1$ Wasserstoff ist und $R_2$ eine der folgenden Gruppen ist

$-CH_2-C_6H_5$

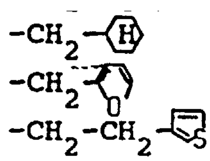

$-CH_2-CH(CH_3)_2$

oder die Gruppe

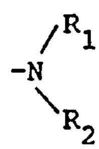

für

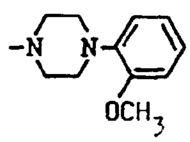

steht.

Von den neuen Verbindungen können die folgenden als bevorzugt hervorgehoben werden:

-) Verbindung, worin $R_7$ und $R_8$ Methoxy sind.

-) Verbindung , worin $R_{12}$ CN, $OCH_3$, $CH_3$, $C_2H_5$, $C(CH_3)_3$, F, Cl oder $CF_3$ ist.

-) Verbindung, worin $-NR_1R_2$ die Gruppe

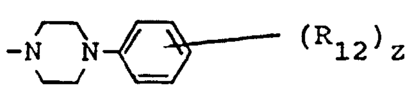

ist, worin $R_{12}$ und z wie oben definiert sind.

-) Verbindung, worin $R_7$ und $R_8$ Methoxy sind und $-NR_1R_2$ für eine der folgenden Gruppen steht:

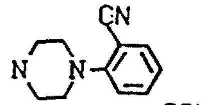

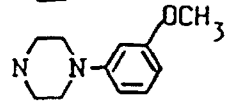

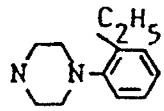

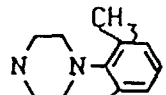

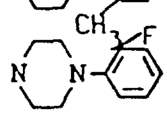

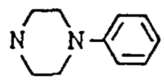

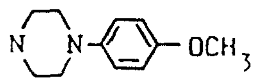

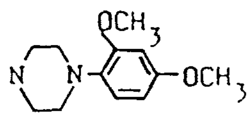

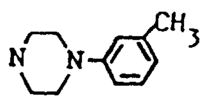

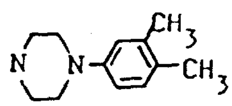

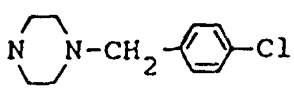

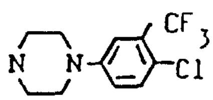

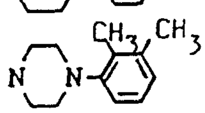

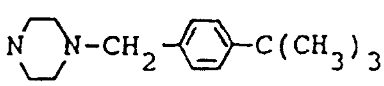

-) Verbindung , worin $NR_1R_2$ für

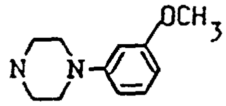

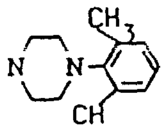

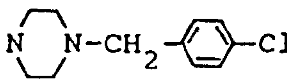

steht.

-) Verbindung der allgemeinen Formel I

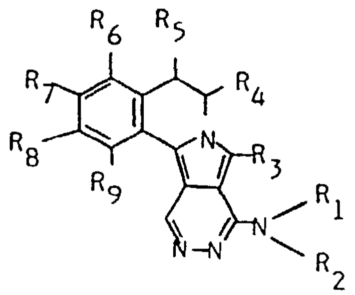

I

worin $R_3$, $R_4$, $R_5$, $R_6$ und $R_9$ Wasserstoff sind, $R_7$ und $R_8$ Alkoxy mit 1 - 4 Kohlenstoffatomen sind oder $R_7$ und $R_8$ zusammen $-OCH_2O-$ oder $-OCH_2CH_2O-$ sind und die Gruppe $-NR_1R_2$ für

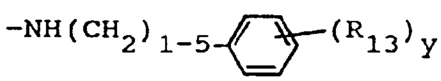

oder

$-NH(CH_2)_1$ oder $_2CH(C_6H_5)_2$ steht, worin $R_{13}$ $CF_3$, $C(CH_3)_3$ oder $-OCH_2C_6H_5$ ist und y 1 oder 2 ist, oder deren physiologisch verträgliche Salze mit Säuren, Basen oder Komplexbildnern.

-) Verbindung , worin $-NR_1R_2$ die Gruppe

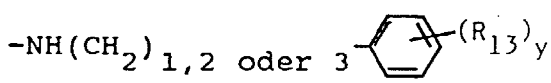

bedeutet, worin $R_{13}$ und y wie oben definiert sind.

-) Verbindung, worin $R_7$ und $R_8$ Methoxy sind.

-) Verbindung, $R_7$ und $R_8$ Methoxy sind und $-NR_1R_2$ eine der folgenden Bedeutungen hat:

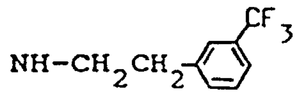

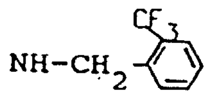

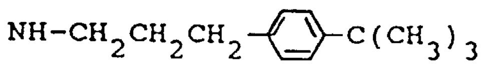

$NH-CH_2-CH_2-CH(C_6H_5)_2$

$NH-CH_2-CH(C_6H_5)_2$

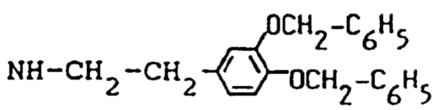

Verbindung der allgemeinen Formel I

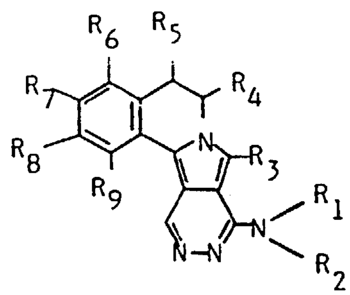

I

worin $R_3$, $R_4$, $R_5$, $R_6$ und $R_9$ Wasserstoff sind, $R_7$ und $R_8$ Methoxy sind und $-NR_1R_2$ eine der folgenden Bedeutungen hat:

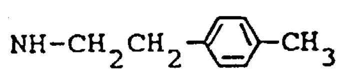

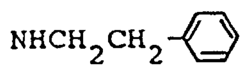

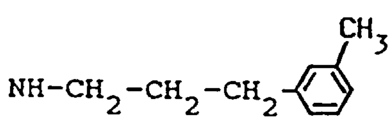

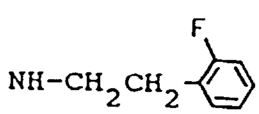

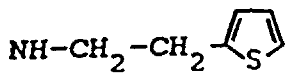

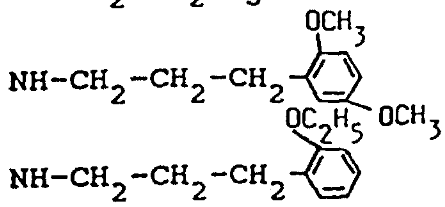

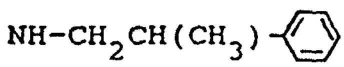

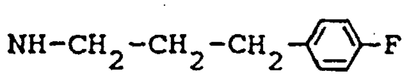

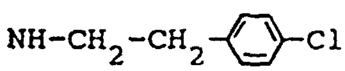

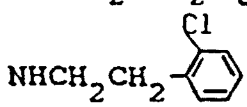

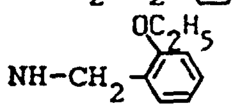

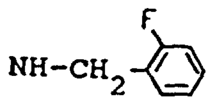

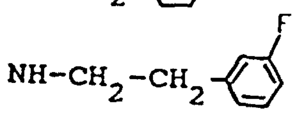

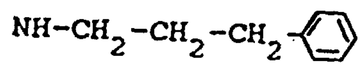

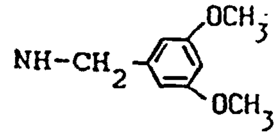

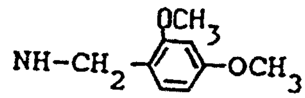

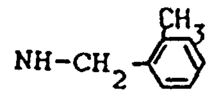

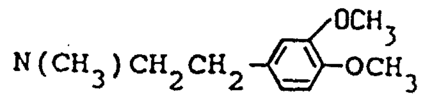

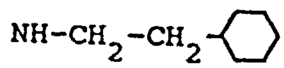

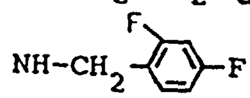

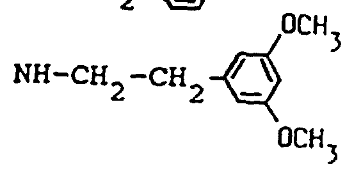

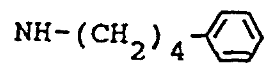

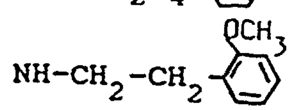

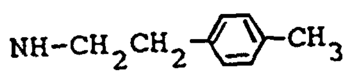

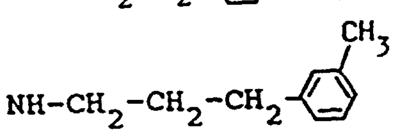

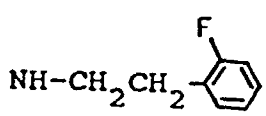

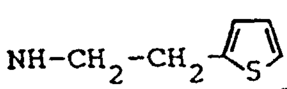

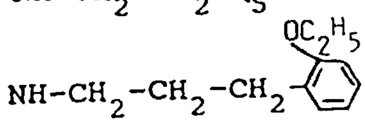

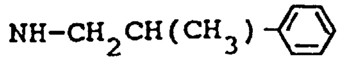

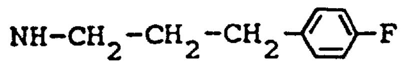

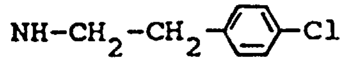

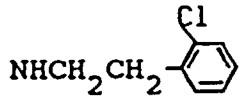

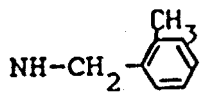

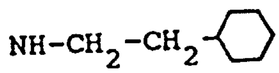

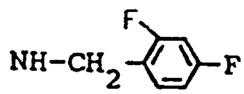

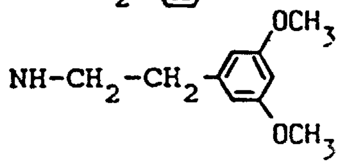

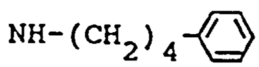

vorzugsweise

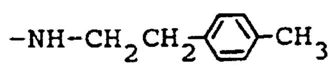

ist.

In den folgenden Tabellen 1 und 2 werden Beispiele von Verbindungen der Formel I zusammengefaßt.

Die Verbindungen der Tabelle 1 sind aus den bereits genannten Veröffentlichungen DE 35 00 941, DE 35 25 048, EP 190 563 und EP 252 299 bekannt.

Die Verbindungen der Tabelle 2 sind Beispiele der neuen Verbindungen der allgemeinen Formel I.

Tabelle 1

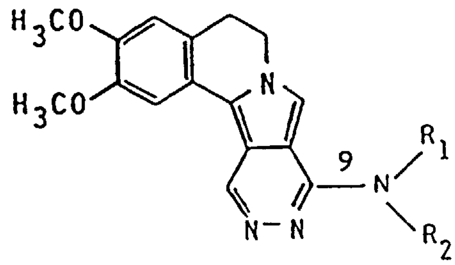

| 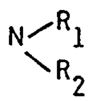 | Salzform |
|---|---|
| $NH-CH_2CH_2N(CH_3)_2$ | HCl |
| $NH-NH_2$ | HCl |
| $N(CH_3)_2$ | HCl |
| $NH_2$ | Base |
| $NHCH_3$ | HCl |
| $NHC_2H_5$ | HCl |
| $N(C_2H_5)_2$ | HCl |
| NH- 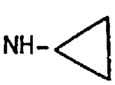 | HCl |
| 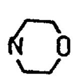 | HCl |
| $NH-CH_2-CH(CH_3)_2$ | HCl |
| $NH-CH_2-C_6H_5$ | HCl |

| 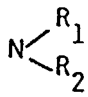 | Salzform |
|---|---|
| $NH-CH(CH_3)_2$ | HCl |
| $NH-CH_2-CH_2-CH_2-CH_3$ | HCl |
| $NH-N(CH_3)_2$ | HCl |
| $NH-CH_2-CH_2-OCH_3$ | HCl oder HJ |
| 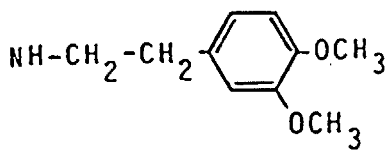 | HCl |
| 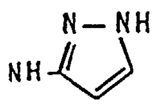 | HCl |
| 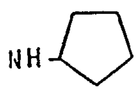 | HCl |
| 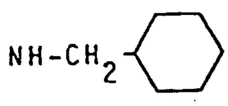 | HCl |
| $NH-CH_2-C \equiv CH$ | HCl |
| 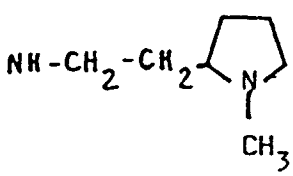 | HCl |
| 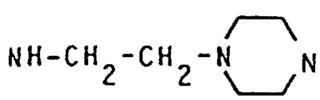 | HCl |
| 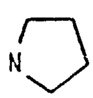 | HCl |
| $NH-CH_2-CH_2-CH_3$ | HCl |

| 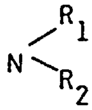 | Salzform |
|---|---|
| $NH-CH_2-CH=CH_2$ | HCl |
| 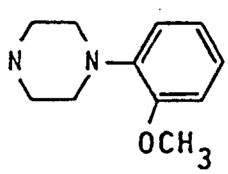 | HCl |
| $NH-CH_2-CH_2-CH_2-N(CH_3)_2$ | HCl |
| 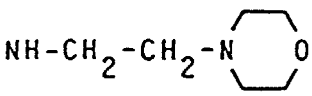 | HCl |
| $NH-CH_2-CH_2-CH_2-CH_2-CH_3$ | HCl |
| 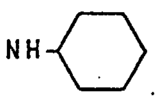 | HCl |
| 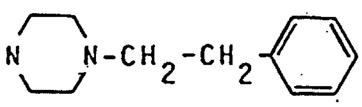 | HCl |
| 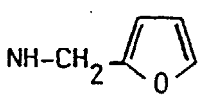 | HCl |

| 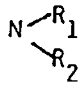 | Salzform |
|---|---|
| $NH-CH_2-CH_2-CH_2-OCH_3$ | HCl |
| $NH-CH_2-CH_2-CH(CH_3)_2$ | HCl |
| 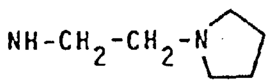 | HCl |
| $NH-CH_2-CH_2-OH$ | HCl |
| $NH-N=C(CH_3)_2$ | Base |
| $NH-C_6H_5$ | HCl |
| 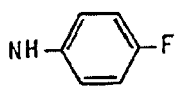 | |
| 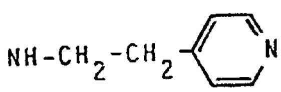 | HCl |
| 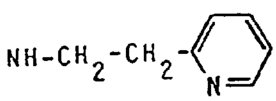 | HCl |
| 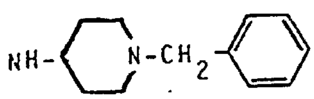 | HCl |
| 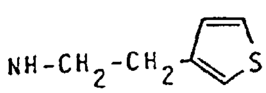 | HCl |
| 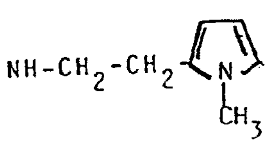 | HCl |
| 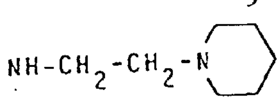 | HCl |

Tabelle 2

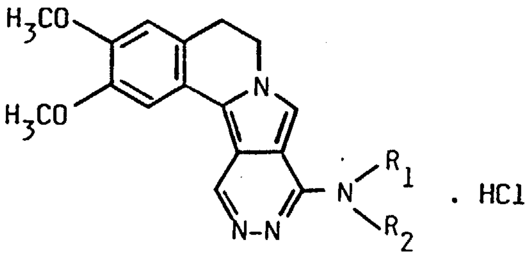

| 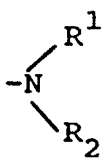 | Fp °C |
|---|---|
| 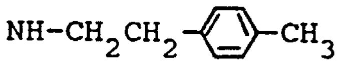 | 270-272 |
| 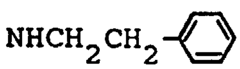 | 274-275 |
| 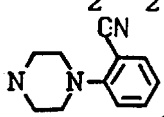 | 291 |
| 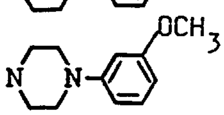 | 266-268 |
| 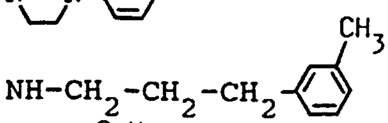 | 243-245 |
| 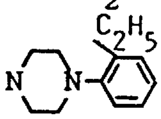 | 305 |
| 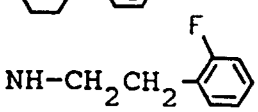 | 268-269 |
| 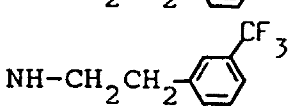 | 264-266 |

| 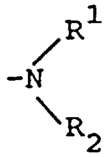 | Fp °C |
|---|---|
| 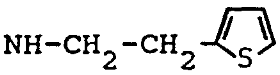 | 277 |
| 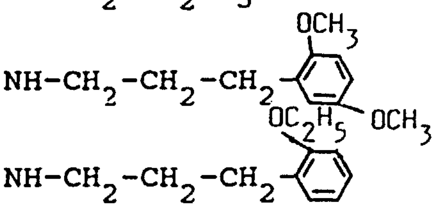 | 210-211 |
| | 269-270 |
| 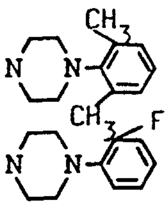 | 294-295 |
| | 295 |
| 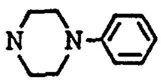 | 289-290 |
| 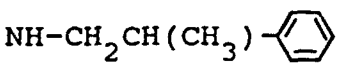 | 252-253 |
| 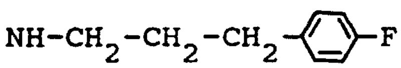 | 250-251 |
| 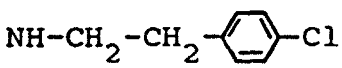 | 293-295 |
| 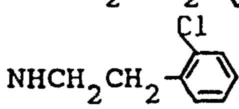 | 266-267 |
| 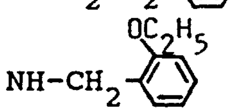 | 176-178 |
| 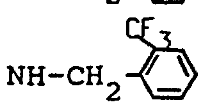 | 289-290 |
| 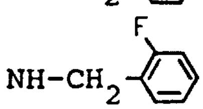 | 272-273 |
| 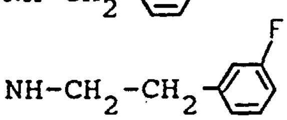 | 278 |

| 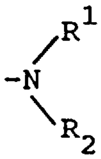 | Fp °C |
|---|---|
| 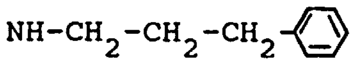 | 253-254 |
| 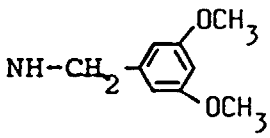 | 225 |
| 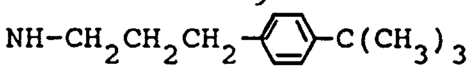 | 269-270 |
| 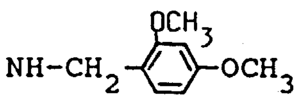 | 241-243 |
| 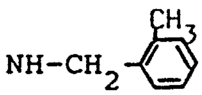 | 268-269 |
| $NH-CH_2-CH_2-CH(C_6H_5)_2$ | 180-182 |
| 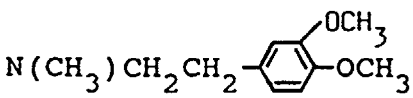 | 244 |
| 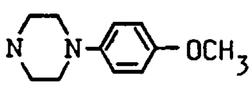 | 284-285 |
| 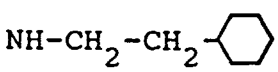 | 281-282 |
| 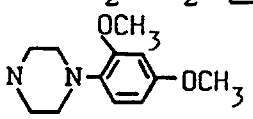 | 262-263 |
| 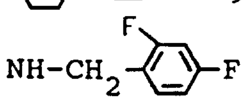 | 266-268 |
| 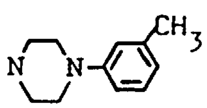 | 285 |
| $NH-CH_2-CH(C_6H_5)_2$ | 296 |
| 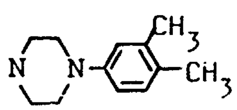 | 274 |

| 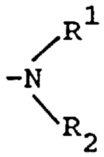 | Fp °C |
|---|---|
| 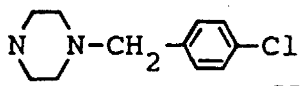 | 229-231 |
| 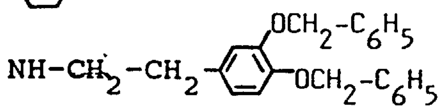 | 154-156 |
| 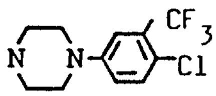 | 288 |
| 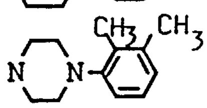 | 285-286 |
| 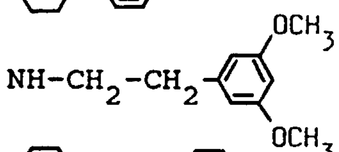 | 247 |
| 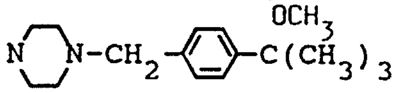 | 298 |
| 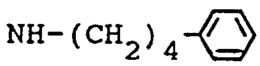 | 240 |
| 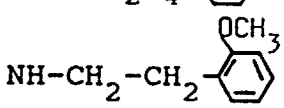 | 222 |

Aus den deutschen Patentanmeldungen DE 35 00 941.1, DE 35 25 048.8 und dem europäischen Patent 190 563 sind cardiotonisch wirksame Verbindungen der allgemeinen Formel I bekannt geworden. Diese Verbindungen sind gemäß diesen Veröffentlichungen für die Bekämpfung von Herzinsuffizienz und/oder cerebralen Stoffwechselstörungen zu verwenden. Aus der europäischen Patentanmeldung Nr. 252 299 (A) ist bekannt geworden, daß diese Verbindungen cardio- und neuroprotektive Wirkung besitzen und darüber hinaus die Gewebsdurchblutung und Gewebssauerstoffversorgung im zentralen Nervensystem fördern.

Ein Aspekt der vorliegenden Erfindung sind die oben angegebenen neuen Verbindungen und diese Verbindungen enthaltende pharmazeutischen Zubereitungen. Gegenstand der vorliegenden Erfindung ist ferner die Verwendung dieser neuen Verbindungen. Die Verbindungen sind zur Behandlung von degenerativen und nekrotisierenden Erkrankungen des Gehirns vorteilhaft. Ebenso ist die vorbeugende Behandlung von durch solche Krankheiten gefährdeten Patienten möglich. Diese Wirkung der Verbindungen beruht nicht auf einer Verbesserung der Gewebsdurchblutung. Die Verbindungen sind somit für eine neuartige Behandlung von Epilepsie und der Alzheimer-Krankheit geeignet, und insbesondere bei der Behandlung von Patienten, die einen Schlaganfall erlitten haben oder gefährdet sind, einen Schlaganfall zu erleiden.

Gegenstand der vorliegenden Erfindung ist ferner die Verwendung der genannten alten und neuen Verbindungen der allgemeinen Formel I und deren Salzen für die Herstellung von Mitteln zur Behandlung chronisch inflammatorischer Prozesse, der Colitis Ulcerosa und des Morbus Crohn und von Mitteln mit antiproliferativer Wirkung. Die Wirkung der Verbindungen kann durch ihre Hemmung unselektiver Kationenkanäle (UKK) erklärt werden.

Der Pathophysiologie des chronischen Bronchialasthma liegen entzündliche Prozesse zugrunde, die durch die Aktivierung inflammatorischer Zellen vermittelt werden. (BARNES, 1987; SEIFERT und SCHULTZ, 1991).

Die rezeptor-regulierte Aktivierung inflammatorischer Zellen (z.B. neutrophile Granulozyten und Mastzellen bzw. deren permanente Zellinien HL-60 Zellen oder sensibilisierte, d.h. Gammaglobulin E beladene RBL-Zellen) wird unabhängig von der Art der stimulierenden Agonisten (z.B. Endothelin, PAF, Leukotriene, chemotaktisches Peptid fMLP oder Antigen gegen sensibilisierte Mastzellen) durch Blocker unselektiver Kationenkanäle (UKK) inhibiert (RINK, 1990). Durch diese Kanäle gelangt extrazelluläres Calcium in die Zellen, das für die Persistenz der rezeptor-vermittelten

Zellaktivierungen erforderlich ist (PUTNEY, 1990). Wird diese Calciumzufuhr unterbrochen resultiert eine Blockade der Aktivierunginflammatorischer Zellen.

Klassische Calciumantagonisten vom Dihydropyridin- bzw. Phenylalkylamin-Typ hemmen weder UKKs noch inflammatorische Prozesse (WELLS et al., 1986).

Als Maß der Zellaktivierung bzw. als Maß von deren Hemmung durch UKK-Blocker wird fluorometrisch die Kinetik der cytoplasmatischen Calciumionenkonzentration in Fura-2-beladenen Zellen anhand der von GRYNKIEWICZ et al. (1985) beschriebenen Methode quantifiziert. Diese Vorgehensweise hat sich im Rahmen dieser Erfindung als zuverlässige Screeningmethode zur Auffindung von UKK-Blockern erwiesen.

Zur spezifischen Charakterisierung von Blockern unselektiver Kationenkanäle eignet sich die sogenannte funktionelle THAPSIGARGIN-Inhibition. THAPSIGARGIN ist ein von THASTRUP et al. (Proc. Natl. Acad. Sci. (USA), 87, 2466-2470, 1990) beschriebener Tumorpromotor, der selektiv und irreversibel die $Ca^{2+}$ATPase intrazellulärer, $IP_3$-sensitiver $Ca^{2+}$-Speicher hemmt. Infolge dessen kommt es zu einer raschen Entleerung der $Ca^{2+}$-Speicher. Wie von J. PUTNEY (Calcium, 11, 611-624, 1990) beschrieben stellt die Entleerung dieser Speicher den physiologischen Reiz für die Öffnung unselektiver Kationenkanäle in der Zellmembran dar. Die Folge ist ein massiver Einstrom von $Na^+$ und $Ca^{2+}$ in die Zelle. Aufgrund dieser Eigenschaften eignet sich Thapsigargin als indirekter Stimulator zur agonisten- und $IP_3$-unabhängigen Öffnung der unselektiven Kationenkanäle.

Im Rahmen der vorliegenden Erfindung wurde die Thapsigargin-Stimulation unselektiver Kationenkanäle an HL 60 Zellen (menschliche Leukämiezelle), an hippocamoalen und corticalen Neuronenzellen sowie an RBL-Zellen (rat basophilic lymphoma ) erfolgreich durchgeführt und somit wurde die Existenz dieser Kanäle in den jeweiligen Zellinien nachgewiesen.

Die zytoplasmatische $Ca^{2+}$Konzentration ($[Ca^{2+}]_i$) spielt eine wichtige Rolle bei der Zellproliferation und beim Tumorwachstum (Übersicht siehe L.R. ZACHARSKI, Journal of Medicine 19: 145-177, 1988). Insbesondere der durch Rezeptoraktivierung mit konsekutiver Inositoltrisphosphat-($IP_3$-)-Vermittlung stimulierte $Ca^{2+}$-Einstrom in die Zelle soll von entscheidender Bedeutung sein für die oncogene Zellproliferation (U. KIKKAWA und Y. NISHIZUKA, Ann. REV. CELL. BIOL. 2: 149-178, 1986). Dieser Mechanismus spielt auch eine Rolle bei der Metastasenbildung und der "Multi-Drug-Resistance". (Übersicht siehe die obengenannten Veröffentlichung von L.R. ZACHARSKI.) J. MED. 19: 145-177, 1988.

Diese Hypothese wird dadurch gestützt, daß Thapsigargin als indirekter Stimulator unselektiver Kationenkanäle (UKK) sowohl zu einem $Ca^{2+}$-overload in der Zelle führt als auch ein hochwirksamer Tumorpromotor ist.(V. THASTRUP et al. Proceedings of the NATL. Acad. Sci: (USA) 87: 2466-2470, 1990.)

Die Blockade des $Ca^{2+}$-Einstroms durch die UKK führt zu einer Normalisierung der intrazellulären Ca-lonenkonzentration und somit zu einer Hemmung des Tumorwachstums etc.

Klassiche Calciumantagonisten hemmen diese UKK nicht. Überraschend ist festgestellt worden, daß die Verbindungen dieser Erfindung den Calciumeinstrom in die Zelle durch die UKK hemmen.

Wie von S. H. MURCH et al. (Lancet 339 : 381-385, 15. Febr. 1992) gezeigt wurde, spielt Endothelin I eine wichtige pathophysiologische Rolle bei entzündlichen Darmerkrankungen wie der Colitis ulcerosa und des Morbus Crohn. Mit Hilfe immunhistochenischer Methoden konnte festgestellt werden, daß Patienten mit M. Crohn im Bereich der Submucosa und Patienten mit Colitis ulcerosa im Bereich der Lamina propria des Dickdarmepithels signifikant und stark erhöhte Endothelin I Konzentrationen im Vergleich zu gesunden Normalpersonen aufweisen. Es wird angenommen, daß die lokale Ausschüttung von Endothelin massive Vasospasmen mit konsekutiver disseminierter Ischämie mit Mikroinfarkten hervorruft, die als eigentliche Ursache der genannten Erkrankungen angesehen werden. Die vasospasmogene Effektivität des Endothelins wird über einen $Ca^{2+}$-overload vaskulärer Myozyten erklärt. Dabei löst Endothelin primär eine $IP_3$-vermittelte intrazelluläre $Ca^{2+}$-Freisetzung aus, an die sich ein massiver transmembranärer $Ca^{2+}$-Einstrom durch dihydropyridin-insensitive Kanäle anschließt. (M. S. Simönson et al. Clin. Invest. Med. 14: 499-507, 1991; T. Masakai, J. Cardiovasc. Pharmacol. 13:Suppl. 5, S1-S4, 1989; D. W. Hay, R. J. Pharmacol. 100: 383-392, 1990) Bei diesen Kanälen handelt es sich um unselektive Kationen Kanäle, deren Existenz kürzlich auch in Zellen der Dickdarmmukosa beschrieben wurde. (Chr. Siemer und H. Gögelein, Europ. J. Physiol. 420: 319-328, 1992).

Als geeignetes Screening Modell zur Auffindung funktioneller Endothelinantagonisten hat sich die Endothelin stimulierte Aktivierung Fura-2 beladener menschlicher Leukämiezellen (HL 60 Zelle) bewährt. In Anlehnung an G. GRYNKIEWICZ et al. (J. Biol. Chem. 260:340-3450, 1985)läßt sich die intrazelluläre $Ca^{2+}$-Konzentration im Cytoplasma von HL 60 Zellen (Suspensionen) spektrofluorometrisch verfolgen und als Maß der Zellaktivierung durch Endothelin quantifizieren. Die Stimulation erfolgte durch Zusatz von 0.1 µM Endothelin, sie ließ sich dosisabhängig durch die erfindungsgemäßen Substanzen inhibieren.

Der funktionelle Endothelinantagonismus der erfindungsgemäßen Substanzen wird über eine Blockade der unselektiven Kationen Kanäle vermittelt. Deshalb eignet sich auch der Nachweis eines funktionellen Thapsigargin-Antagonismus an RBL-hml-Zellen als Screening Methode für funktionelle Endothelinantagonisten.

Ausführung der Untersuchung:

Für Screeningzwecke werden in $Ca^{2+}$ freiem Inkubationsmedium Fura-2-beladene adhäsive RBL-hm 1-Zellen mit

0,1 µM Thapsigargin stimuliert. Nach 4 Minuten wird extrazelluläres $Ca^{2+}$ auf 1,5 mM restituiert und anhand der Fura-2-Fluoreszenz der exzessive Anstieg der cytoplasmatischen $Ca^{2+}$-Konzentration infolge eines massiven transmembranären $Ca^{2+}$-Einstroms durch unselektive Kationenkanäle registriert.

Dieser Einstrom ist ausschließlich und dosisabhängig zu inhibieren durch unselektive Kationen-Kanal-Blocker. Weder klassische Kalziumantagonisten noch spezifische Blocker von Agonisten, die den $IP_3$-Turnover stimulieren können den durch Thapsigargin indirekt ausgelösten transmembranären $Ca^{2+}$-Einstrom hemmen. Die Verbindungen der vorliegenden Erfindung zeichnen sich durch eine Hemmung der UKK aus.

Die fluorometrische Calciummessung im Cytoplasma einzelner adhärenter RBL-hml-Zellen erfolgt in Analogie zu der von KUDO und OGURA (1986) für neuronale Zellen beschriebenen Methode. Verwendet wird ein AXIOVERT 35 Fluoreszenzmikroskop von ZEISS in Kombination mit einem Imaging-System von HAMAMATSU, bestehend aus dem ICMS-Bildverarbeitungssystem, Restlichtkamera mit Kontrolleinheit und Bildverstärker DVS 3000.

Die Kinetik der cytoplasmatischen $Ca^{+2}$-Konzentration wird als Konzentrations-Zeit-Kurve nach der durch Thapsigargin (0,1 µM) stimulierten Zellaktivierung fortlaufend aufgezeichnet. Verglichen werden die Kurven zweier aktivierter Zellkulturen in Gegenwart und Abwesenheit von 10 µM Testsubstanz. Die Fläche unter diesen Kurven (area under the curve = AUC) wird integriert und als Maß der Zellaktivierung registriert. Die inhibitorische Wirkungsstärke der getesteten UKK-Blocker wird ermittelt anhand folgender Beziehung:

$$\%H = 100 - \frac{AUC_{inh} \times 100}{AUC_{(Kontrolle)}}$$

%H = die prozentuale Hemmung des Calciumeinstroms durch unselektive Kationenkanäle der stimuliert und durch 10 µM Testsubstanz inhibiert wird.

$AUC_{inh}$ = Fläche unter der Kurve, die in Gegenwart des Stimulans plus 10 µM inhibitorischer Testsubstanz aufgezeichnet wird.

AUC Kontr. = Fläche unter der Kurve, die nur nach Zusatz des Stimulans registriert wird.

Literatur zu den obigen Erläuterungen:

BARNES P.J., I.W. RODGER und N.C. THOMSON
Pathogenesis of asthma, in "ASTHMA, basic mechanisms and clinical management"
ED by P.J. BARNES; ACADEMIC PRESS, LONDON, 1988

GRYNKIEWICZ G., M. POENIE und R.Y. TSIEN
A new generation of $Ca^{2+}$-indicators with greatly improved fluorescence properties
J. BIOL. CHEM. 260: 3440-3450, 1985

HIDE, M. und M.A. BEAVEN
Calcium influx in a rat mast cell (RBL-2H3) line
J. BIOL. CHEM. 266 15221-15229, 1991

KUDO, Y. und A. OGURA
Glutamate-induced increase in intracellular $Ca^{2+}$-concentration in isolated hippocampal neurones
BR. J. PHARMACOL. 89: 191-198, 1986

PUTNEY, J.W., jr.
Capacitative Calcium entry revised
CELL CALCIUM 11: 611-624, 1990

RINK, T.J.
Receptor-mediated calcium entry
FEBS LETT. 268: 381-385, 1990

SEIFERT, R. und G. SCHULTZ
The superoxide forming NADPH oxidase of phagocytes: An enzym system regulated by multiple mechanism
REV. PHYSIOL. BIOCHEM. PHARMACOL., Vol. 117, SPRINGER VERL., 1991

WELLS, E., C.G. JACKSON, S.T. HARPER, J. MANN and R.P. EAOY
Characterization of primate bronchoalveolar mast cells II, inhibition of histamine, $LTC_4$ and $PGF_{2\alpha}$ release from primate bronchoalveolar mast cells and a comparison with rat peritoneal mast cells
J. IMMUNOL. 137: 3941-3945, 1986.

Meßergebnisse:
Angegeben wird die prozentuale Hemmung der UKK nach Thapsigarginstimulation (0,1 µM Thapsigargin) in RBL-hm 1 Zellen. Die Konzentration der Testsubstanzen ist $10^{-5}$ Mol (Tabelle 3) beziehungsweise 1 µ Mol und 10 µ Mol (Tabelle 4).

## Tabelle 3

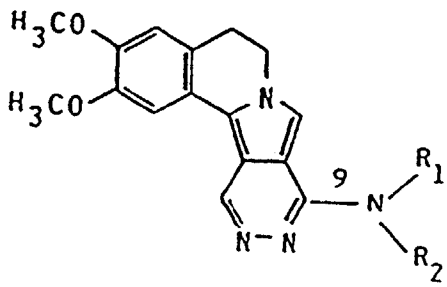

RBL - hm 1 Zellen - Thapsigargin (0,1 µM)-Stimulation

| 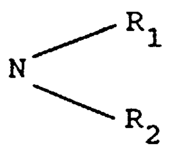 | Salzform | $IC_{50}$ | %H |
|---|---|---|---|
| $NH-CH_2-CH(CH_3)_2$ | HCl | - | 65,7 |
| $NH-CH_2-C_6H_5$ | HCl | $1,8.10^{-6}$ | 90,1 |
| $NH-CH_2-$ 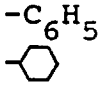 | HCl | $2,5.10^{-6}$ | 90,1 |
| 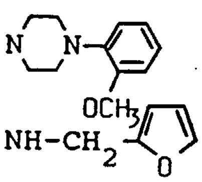 | HCl | $1,77.10^{-6}$ | 88,8 |
| $NH-CH_2-$ | HCl | - | 62,5 |
| $NH-CH_2-CH_2-$ 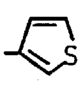 | HCl | $2,37.10^{-6}$ | 88,4 |

Tabelle 4

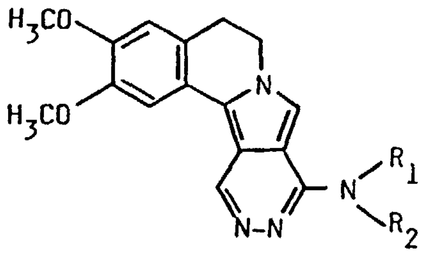 . HCl

RBL - hm 1 Zellen - Thapsigargin (0,1 µM)-Stimulation

(1) Konzentration der Testsubstanz 1 µMol

(2) Konzentration der Testsubstanz 10 µMol

| 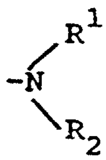 | %H 1 µM(1) | %H 10 µM(2) | $IC_{50}$ |
|---|---|---|---|
| 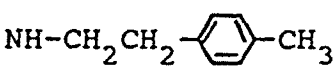 | | 95.36 | $78.10^{-7}$ |
| 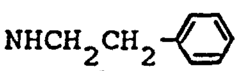 | 31.7 | 87.87 | |
| 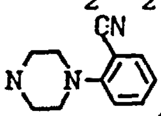 | 41.6 | 77.70 | |
| 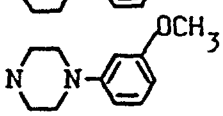 | | 92.34 | $1.5.\ 10^{-6}$ |
| 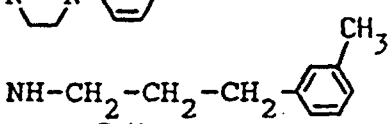 | | 78.67 | |
| 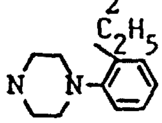 | 34.9 | 82.60 | |
| 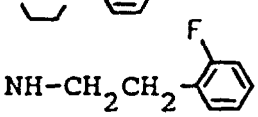 | 51.2 | 81.55 | $1.47.10^{-6}$ |
| 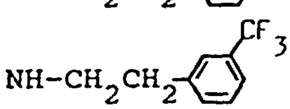 | | 97.94 | $7.1.10^{-7}$ |

| 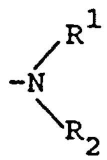 | %H 1 μM(1) | %H 10 μM(2) | $IC_{50}$ |
|---|---|---|---|
| 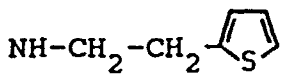 | | 88.41 | |
| 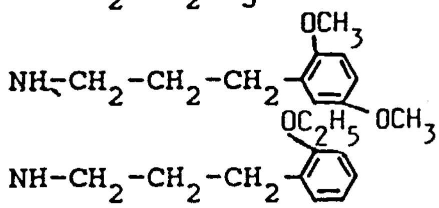 | | | |
| | 48.7 | 96.48 | |
| 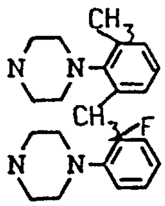 | 38.1 | 96.18 | |
| | 37.9 | 67.37 | |
| 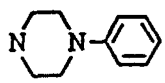 | | 71.94 | |
| 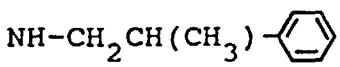 | 32.1 | 89.84 | |
| 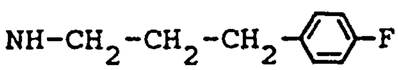 | | 69.25 | |
| 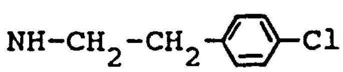 | 56.6 | 96.25 | |
| 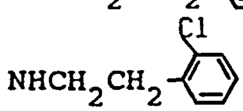 | 50.5 | 98.5 | |
| 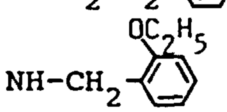 | | | |
| 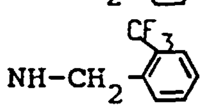 | | | |
| 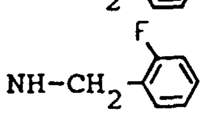 | | | |
| 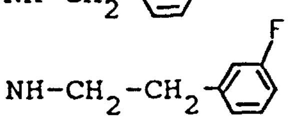 | | | |

| 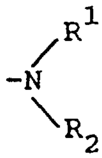 | %H 1 µM(1) | %H 10 µM(2) | $IC_{50}$ |
|---|---|---|---|
| 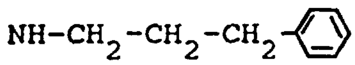 | | | |
| 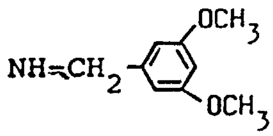 | | | |
| 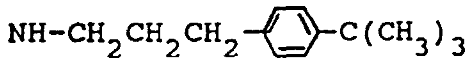 | | 87.20 | |
| 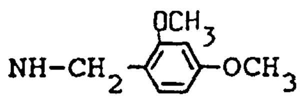 | | | |
| 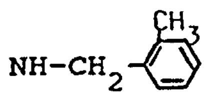 | 50.4 | 88.71 | |
| $NH-CH_2-CH_2-CH(C_6H_5)_2$ | 57.2 | 82.11 | |
| 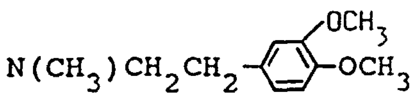 | | 56.35 | |
| 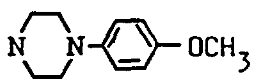 | | 48.86 | |
| 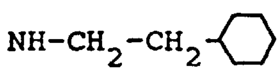 | | 98.14 | |
| 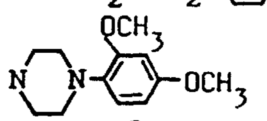 | | 60.50 | |
| 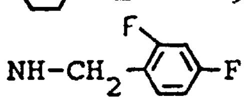 | 37.2 | 83.3 | |
| 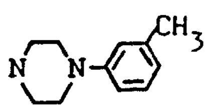 | | 62.64 | |
| $NH-CH_2-CH(C_6H_5)_2$ | | 35.60 | $1.43 \cdot 10^{-6}$ |
| 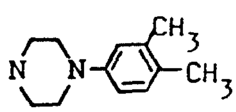 | | 55.84 | |

| 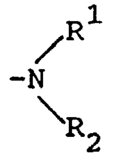 | %H 1 µM(1) | %H 10 µM(2) | $IC_{50}$ |
|---|---|---|---|
| 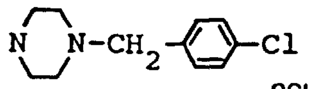 | | 99.40 | 1.43 . $10^{-6}$ |
| 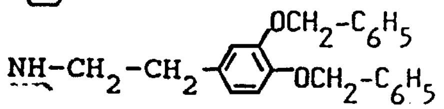 | | 71.99 | |
| 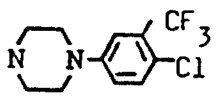 | | 58.80 | |
| 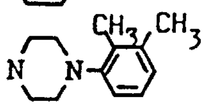 | | 84.50 | |
| 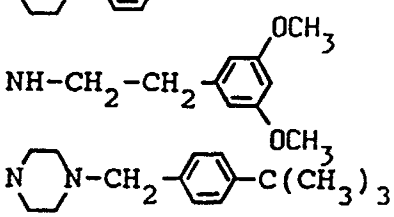 | | 91.98 | |
| | | 75.19 | |
| 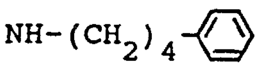 | 35.3 | 93.0 | |
| 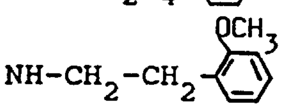 | | | |

Anhand des folgenden Tests kann die funktionelle antiinflammatorische Effektivität gezeigt werden:

Verwendet werden einzelne an Glasplättchen adhärente RBL-2H3-Zellen (eine den Mastzellen verwandte Tumorzellinie).

Die Kultivierung und Anhaftung der RBL-2H3-Zellen erfolgt in der HIDE und BEAVEN (1991) beschriebenen Methode. Zur Sensibilisierung der adhäsiven RBL-2H3-Zellen werden die Zellen 2 Stunden bei Raumtemperatur mit einer 1:2000 verdünnten käuflichen Gammaglobulin E-Lösung gegen einen Dinitrophenol-Rinderserumalbumin-Komplex (DNP-BSA-Antigen) inkubiert. Anschließend werden die Zellen gewaschen. Durch Zusatz von 0,1 ml DNP-BSA-Lösung (10 µg/ml) erfolgt eine massive immunologische Zellaktivierung, die durch einen cytoplasmatischen $Ca^{2+}$-overload vermittelt wird. Die fluorometrische Calciummessung im Cytoplasma einzelner adhärenter RBL-2H3-Zellen erfolgt in Analogie zu der von KUDO und OGURA (1986) für neuronale Zellen beschriebenen - Methode, die auch weiter oben in dieser Beschreibung erläutert wird.

Als Vergleichssubstanz dient in diesen Untersuchungen (10 µM) Chromoglycat, das eine ca. 50 %ige Hemmung der antigen-induzierten Zellaktivierung bewirkt.

In diesem Test zeigen die oben genannten Verbindungen %H Werte, die den oben angegebenen Werten vergleichbar sind.

Tabelle 5

RBL-2H3-Zellen: AK (Monoklonal-Maus) 1:2000
Stimulierung: DNP-BSA (10 $\mu$g/ml).

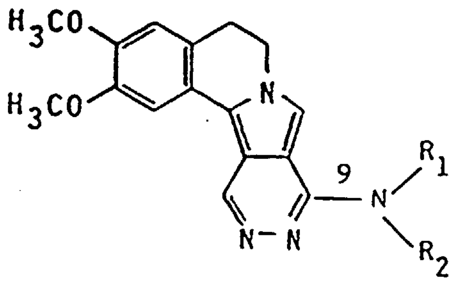

| 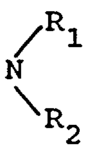 | Salzform | %H |
|---|---|---|
| $NH-CH_2-CH(CH_3)_2$ | HCl | 41,4 |
| $NH-CH_2-C_6H_5$ | BS | 97,9 |
| $NH-CH_2-$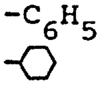 | HCl | 95,4 |
| 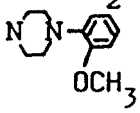 | HCl | 82,0 |
| $NH-CH_2-$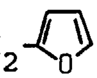 | HCl | 54,4 |
| $NH-CH_2-CH_2-$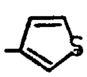 | HCl | 65,8 |
| Fenoterol | | 38,5 |
| Na-Chromo. | | 9,0 |

Untersuchungen an Mikrokulturen verschiedener menschlicher Tumorzellinien mit Hilfe des Tetrazolium Assays zur Feststellung des antiproliferativen Effektes der erfindungsgemäßen Substanzen zeigte sich überraschenderweise, daß die geprüfte Verbindung 5 bis 100 mal wirkungsstärker als die Vergleichssubstanz Verapamil sind.

Die antiproliferative Effektivität der Testsubstanzen wurde mit Hilfe des von MOSMANN (J. IMMUNOL. METH. 65: 55-63, 1983), DENIZOT et al. (J. IMMUNOL. METH. 89: 271-277, 1986) und von J. ELIASON et al. (INT. J. CANCER 46: 113-117, 1990) beschriebenen MTT-Tests bestimmt. (MTT = [3-(4,5-dimethylthiazol-2yl)2,5-diphenyl-tetrazoliumbromid] von CHEMICON Inc. El Segundo, Ca, USA). Dieser Indikator wird nur von lebenden Zellen mit intakten Mitochondrien zu einem blauen Formazan Produkt metabolisiert. Folgende menschliche Tumorzellinien wurden in unserem Test verwendet: A 549 (Adenocarcinom der Lunge), A 431 (Epidermiscarcinom der Vulva), PC 3 (Adenocarcinom der Prostata), SK BR 3 (Adenocarcinom der Mamma), HT-29 (CX1 1) (Adenocarcinom des Colon) und K 562 (chronisch-myeloische Leukämiezelle). Der Test wurde auf Mikrotiterplatten durchgeführt. Jedes Well enthielt 100 $\mu$l einer Zellsuspension (0,2 x $10^6$ Zellen/ml). Als Inkubationsmedium diente RPMI 1640 mit 10% Hitze-inaktiviertem fetalen Kälberserum und 50 $\mu$g/ml Gentamycin. Die Zellsuspensionen wurden 0, 24, 48 oder 72 Stunden bei gesättigter Luft-

feuchtigkeit in einem $CO_2$ (5%) - Luft (95%)-Gemisch bei 37°C inkubiert in Gegenwart und Abwesenheit von variablen Konzentrationen antiproliferativer Testsubstanzen. Die Testsubstanzen wurden in DMSO (Endverdünnung: 0,1 %) gelöst. Anschließend wurden 10 µl MTT-Lösung (3 mg/ml) und nach 3 Stunden 100 µl einer 0,08 N HCl enthaltenden Isopropanollösung zugesetzt. Nach einer weiteren Stunde wurde die Lichtabsorption bei 570 nm (Vergleichswellenlänge 630 nm) in einem Mikroplattenleser ermittelt. Die Lichtabsorption ist direkt proportional zur Anzahl lebender Zellen. Die halbmaximalen Hemmkonzentrationen der untersuchten Substanzen lagen bei 1 µg/ml.

Die vasospasmolytische Effektivität der obengenannten funktionellen Endothelin- bzw. Thapsigargin-Antagonisten wurde am isolierten Gefäßpräparat bestätigt: An retrograd perfundierten spontan schlagenden LANGENDORFF Herzen aus Ratten wurde die coronare Perfusion fortlaufend mittels elektromagnetischer Flußmessung (Apparatur von Hugo Sachs Elektronik, MARCH), quantifiziert. Mit dieser Meßanordnung lassen sich Ausmaß, Dauer und Verlaufsform von Gefäßspasmen mit großer Genauigkeit registrieren. Perfundiert man mit 100 nM Endöthelinkonzentration, so wird der coronare Perfusionsfluß von 11 auf 5 ml/min reduziert. Die Perfusionseinschränkung kann durch die erfindungsgemäßen Substanzen aufgehoben werden. Die Wirkungsstärken der erfindungsgemäßen Verbindungen bezüglich der Thapsigargininhibition an Fura-2-beladenen RBL-hm1-Zellen bzw. der Effektivität der Endothelininhibition an Fura-2 beladenen HL 60 Zellen korrelierte eindeutig mit der am Langendorffpräparat nachgewiesenen vasospasmolytischen Effektivität der untersuchten Substanzen. Es kann daraus geschlossen werden, daß dem vasospasmolytischen Endothelinantagonismus der untersuchten Substanzen eine Blockade unselektiver Kationen Kanäle unterliegt.

Die Verbindungen können sowohl enteral als auch parenteral verabreicht werden. Als Dosis für die orale Anwendung werden 0,1 bis 500 mg Wirkstoff pro Dosis, für die i.v.-Anwendung von 0,05 bis 150 mg pro Dosis vorgeschlagen. Die gewünschte therapeutische Dosis ist von der Indikation und Darreichungsform abhängig und kann experimentell bestimmt werden.

Geeignete Anwendungsformen sind beispielsweise Tabletten, Kapseln, Zäpfchen, Lösungen, Säfte, Emulsionen, Aerosole oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageehüllen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure, hergestellt und in Injektionsflaschen oder Ampullen abgefüllt.

Die eine oder mehrere Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol bzw. dessen Derivaten herstellen.

Verfahren zur Herstellung der Verbindungen der Formel I sind in den europäischen Patentanmeldungen 190 563 und 252 299 beschrieben, auf die hiermit Bezug genommen wird.

Die Verbindungen können erhalten werden durch Umsetzung einer Verbindung der allgemeinen Formel II

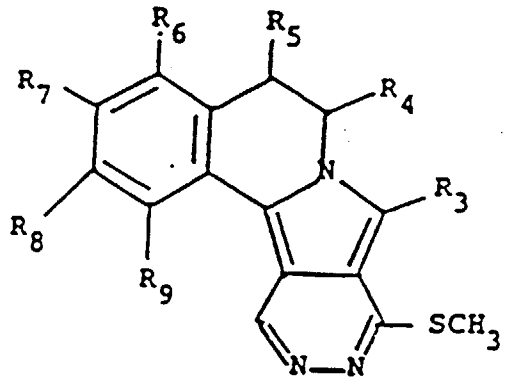

(II)

worin die Reste $R_3$, $R_4$, $R_5$, $R_6$, $R_7$, $R_8$ und $R_9$ die oben angegebene Bedeutung haben, mit einer Verbindung der allgemeinen Formel III

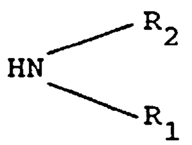

(III)

worin $R_1$ und $R_2$ die oben angegebene Bedeutung haben.

Hierbei wird eine Ausgangsverbindung der allgemeinen Formel II in einem hochsiedenden inerten Lösungsmittel, beispielsweise Dimethylformamid, Dimethylacetamid, Chlorbenzol oder Hexamethylphosphorsäuretrisamid gelöst und mit der Aminkomponente der allgemeinen Formel III unter Rückfluß bis zur Beendigung der Reaktion erhitzt. Die Reaktionszeit beträgt dabei zwischen ca. 1 und 15 Stunden und ist abhängig von den verwendeten Ausgangskomponenten.

Bei reaktionsfähigen Aminen können auch Alkohole oder Tetrahydrofuran als Lösungsmittel verwendet werden; unter Umständen kann auch eine Umsetzung im Autoklav vorteilhaft sein.

Wenn die eingesetzten Amine flüssig und genügend hoch siedend sind, kann die Reaktion auch in einem Überschuß des Amins ohne zusätzliches Lösungsmittel erfolgen (z.B. bei Anilin, Morpholin, Phenylethylamin), gegebenenfalls unter Stickstoffatmosphäre.

In einigen Fällen kann auch ein Reaktionspartner verwendet werden, der bei der Umsetzung sowohl als Lösungsmittel dient, als auch durch Spaltung während der Reaktion das benötigte Amin liefert, z.B. Diemethylformamid.

Die erfindungsgemäßen 9-Amino-pyridazino-pyrazolo-isochinoline sind Basen und können auf übliche Weise mit anorganischen oder organischen Säuren in beliebige physiologisch unbedenkliche Säureadditionssalze übergeführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Citronensäure, Apfelsäure, Benzoesäure, p-Hydroxybenzoesäure, p-Aminobenzoesäure, Phthalsäure, Zimtsäure, Salicylsäure, Ascorbinsäure und Methansulfonsäure.

<u>Beipiele</u>

5,6-Dihydro-2,3-dimethoxy-9-4-[(2,6-dimethyl) piperazino]-pyridazino[4',5':3,4]pyrrolo[2,1-a]isochinol in-hydrochlorid

3 g S-Methylverbindung (Hydrochlorid), 5 ml 1-(2,6-dimethylphenyl)-piperazin und 50 ml Toluol werden ca. 5 h am Rückfluß erhitzt. Nach beendeter Umsetzung (DC-Kontrolle) wird abgekühlt und das Umsetzungsprodukt abgesaugt.

Es wird 2 mal mit Toluol gewaschen, zwischen $CH_2Cl_2$ und verdünnter NaOH verteilt. Die organische Phase wird mehrmals mit Wasser gewaschen, über $Na_2SO_4$ getrocknet und eingedampft. Der Rückstand wird gegebenenfalls nach einer Reinigung über eine Kieselgelsäule (Eluens $CH_2Cl_2$ / NaOH = 100 + 10 V.V.) in wenig $CH_2Cl_2$ aufgenommen und durch Zugabe von ethanol. HCl in das Hydrochlorid überführt.
Ausbeute 2,86 g (68 % d. Th.), Fp: 294-295°C

Pharmazeutische Anwendungsbeispiele

| a) | Dragees | |
|---|---|---|
| | 1 Drageekern enthält: | |
| | Wirkstoff der allgemeinen Formel I | 30,0 mg |
| | Milchzucker | 100,0 mg |
| | Maisstärke | 75,0 mg |
| | Gelatine | 3,0 mg |
| | Magnesiumstearat | 2,0 mg |
| | | 210,0 mg |

Herstellung

Die Mischung der Wirksubstanz mit Milchzucker und Maisstärke wird mit einer 10%-igen wässerigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wässerigen Suspension von Zucker, Titandioxyd, Talkum und Gummi arabicum aufgebracht wird. Die fertigen Dragees werden mit Bienenwachs poliert.

| b) | Tabletten | |
|---|---|---|
| | Wirkstoff der allgemeinen Formel I | 30,0 mg |
| | Milchzucker | 100,0 mg |
| | Maisstärke | 70,0 mg |
| | löslich Stärke | 7,0 mg |
| | Magnesiumstearat | 3,0 mg |
| | | 210,0 mg |

Herstellung

Wirkstoff und Magnesiumstearat werden mit einer wässerigen Lösung der löslichen Stärke granuliert, das Granulat getrocknet und innig mit Milchzucker und Maisstärke vermischt. Das Gemisch wird sodann zu Tabletten von 210 mg Gewicht verpreßt.

| c) | Kapseln | |
|---|---|---|
| | Wirkstoff gemäß Anspruch 1 | 20,0 mg |
| | Milchzucker | 230,0 mg |
| | Maisstärke | 40,0 mg |
| | Talk | 10,0 mg |
| | | 300,0 mg |

Herstellung

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

**Patentansprüche**

**1.** Verwendung von Verbindungen der Formel (I)

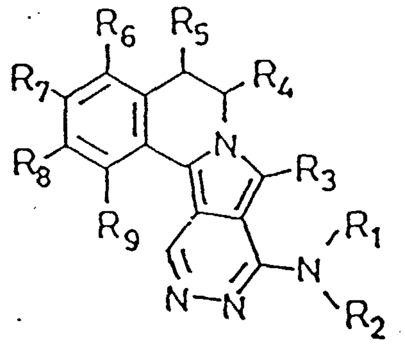

(I)

in der

$R_1$ und $R_2$, die gleich oder verschieden sein können, Wasserstoff; $C_3$-$C_7$ Cycloalkyl; $C_2$-$C_5$ Alkenyl; Phenyl (wobei der Phenylring gegebenenfalls ein- oder zweifach durch Halogen oder Methoxy substituiert ist); Propargyl; einen geradkettigen oder verzweigten, gesättigten oder ungesättigten Alkylrest mit 1 - 5 Kohlenstoffatomen, der substituiert sein kann durch Hydroxy, Alkoxy mit 1 - 4 Kohlenstoffatomen, Halogen, $NH_2$, NH-Alkyl mit 1 - 2 Kohlenstoffatomen, N,N-Di($C_1$-$C_2$)alkylamino, NH-Acyl mit 2 - 4 Kohlenstoffatomen, Cycloalkyl mit 3 - 7 Kohlenstoffatomen, 1 oder 2 Phenylgruppen (wobei der/die Phenylring(e) seinerseits (ihrerseits) ein- oder zweifach substituiert sein kann (können) durch Halogen, $CF_3$, Alkyl mit 1 - 4 Kohlenstoffatomen, Alkoxy mit 1 - 2 Kohlenstoffatomen, NH-Alkyl mit 1 - 2 Kohlenstoffatomen, N,N-Dialkyl mit 1 - 2 Kohlenstoffatomen, $NH_2$, N-Acyl mit 2 - 3 Kohlenstoffatomen, Alkylsulfonylamino oder Benzyloxy), Furyl, Thienyl, einen stickstoffhaltigen heterocyclischen 5- oder 6-Ring der gegebenenfalls als weiteres Heteroatom ein Sauerstoff- oder Schwefelatom enthalten kann (wobei der Ring gegebenenfalls durch Alkyl mit 1 - 4 Kohlenstoffatomen substituiert ist) bedeuten; oder

$R_1$ und $R_2$ zusammen mit dem Stickstoffatom einen 3- bis 7-gliedrigen Ring, der gegebenenfalls als weiteres Heteroatom ein Sauerstoff- oder ein Stickstoffatom enthalten kann, wobei dieser Ring gegebenenfalls durch Phenyl-($C_0$-$C_4$)-Alkyl substituiert ist (wobei der Phenylring seinerseits ein- oder zweifach durch Halogen, $CF_3$, ($C_1$-$C_4$)Alkoxy, ($C_1$-$C_4$)Alkyl oder CN substituiert sein kann, wobei die Substituenten gleich oder verschieden voneinander sein können) bedeuten; oder

$R_2$, falls $R_1$ Wasserstoff bedeutet, auch $-NH_2$; Di($C_1$-$C_2$) Alkylamino; Acetonylamino; -NH($C_2$-$C_3$)Acyl; einen Alkylsulfonyl- oder Alkoxycarbonylrest mit je 1 - 3 Kohlenstoffatomen in der Alkylkette; die Isopropylidenaminogruppe

( 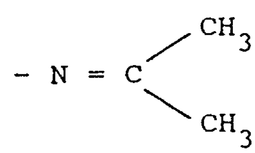 )

oder einen heterocyclischen, ein Stickstoffatom und gegebenenfalls als weiteres Heteroatom ein Sauerstoff-, Stickstoff- oder Schwefelatom enthaltenden 5- oder 6-Ring bedeuten kann;

$R_3$, $R_4$ und $R_5$, die gleich oder verschieden sein können, Wasserstoff oder einen Alkylrest mit 1 - 4 Kohlenstoffatomen bedeuten;

$R_7$ und $R_8$, die gleich oder verschieden sein können, Hydroxy; Alkoxy mit 1 - 4 Kohlenstoffatomen; oder Alkylthio mit 1 - 4 Kohlenstoffatomen bedeuten und

$R_6$ und $R_9$, die gleich oder verschieden sein können, Wasserstoff; Hydroxy; Alkoxy mit 1 - 4 Kohlenstoffatomen; Alkylthio mit 1 - 4 Kohlenstoffatomen; oder den Rest

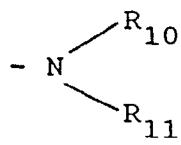

bedeuten, in dem

$R_{10}$ Wasserstoff; oder Alkyl mit 1 - 4 Kohlenstoffatomen ist und
$R_{11}$ Wasserstoff; oder Alkyl mit 1 - 4 Kohlenstoffatomen, wobei der Alkylrest gegebenenfalls durch Hydroxy, Methoxy oder Furfuryl substituiert sein kann;

oder 2 benachbarte Substituenten der Substituenten $R_6$, $R_7$, $R_8$ und $R_9$ zusammen die Gruppe $-O-(CH_2)_{1\text{ oder }2}-O-$ bilden und die jeweils übrigen 2 Substituenten wie oben definiert sind;

sowie deren physiologisch verträglichen Salzen mit Säuren oder Komplexbildnern zur Herstellung von Mitteln zur Behandlung chronisch inflammatorischer Prozesse, der Colitis Ulcerosa und des Morbus Crohn, und von Mitteln mit antiproliferativer Wirkung.

**2.** Verwendung einer Verbindung der Formel (I) nach Anspruch 1, in der

$R_1$ und $R_2$, die gleich oder verschieden sein können, Wasserstoff; $C_3$-$C_7$ Cycloalkyl; $C_2$-$C_5$ Alkenyl; Phenyl (wobei der Phenylring gegebenenfalls ein- oder zweifach durch Halogen oder Methoxy substituiert ist); Propargyl; einen geradkettigen oder verzweigten, gesättigten oder ungesättigten Alkylrest mit 1 - 5 Kohlenstoffatomen, der substituiert sein kann durch Hydroxy, Alkoxy mit 1 - 4 Kohlenstoffatomen, Halogen, $NH_2$, NH-Alkyl mit 1 - 2 Kohlenstoffatomen, N,N-Di($C_1$-$C_2$)alkylamino, NH-Acyl mit 2 - 4 Kohlenstoffatomen, Cycloalkyl mit 3 - 7 Kohlenstoffatomen, Phenyl (wobei der Phenylring seinerseits ein- oder zweifach substituiert sein kann durch Halogen, Alkyl mit 1 - 2 Kohlenstoffatomen, Alkoxy mit 1 - 2 Kohlenstoffatomen, NH-Alkyl mit 1 - 2 Kohlenstoffatomen, N,N-Dialkyl mit 1 - 2 Kohlenstoffatomen, $NH_2$, N-Acyl mit 2 - 3 Kohlenstoffatomen oder Alkylsulfonylamino), Furyl, Thienyl, einen stickstoffhaltigen heterocyclischen 5- oder 6-Ring der gegebenenfalls als weiteres Heteroatom ein Sauerstoff- oder Schwefelatom enthalten kann (wobei der Ring gegebenenfalls durch Alkyl mit 1 - 4 Kohlenstoffatomen substituiert ist) bedeuten; oder

$R_1$ und $R_2$ zusammen mit dem Stickstoffatom einen 3- bis 7-gliedrigen Ring, der gegebenenfalls als weiteres Heteroatom ein Sauerstoff- oder ein Stickstoffatom enthalten kann, wobei dieser Ring gegebenenfalls durch Phenyl-($C_0$-$C_4$)-Alkyl substituiert ist (wobei der Phenylring seinerseits ein- oder zweifach durch Halogen oder Methoxy substituiert ist) bedeuten; oder

$R_2$, falls $R_1$ Wasserstoff bedeutet, auch $-NH_2$; Di($C_1$-$C_2$) Alkylamino; Acetonylamino; -NH($C_2$-$C_3$)Acyl; einen Alkylsulfonyl- oder Alkoxycarbonylrest mit je 1 - 3 Kohlenstoffatomen in der Alkylkette; die Isopropylidenaminogruppe

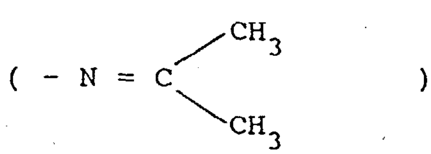

oder einen heterocyclischen, ein Stickstoffatom und gegebenenfalls als weiteres Heteroatom ein Sauerstoff-, Stickstoff- oder Schwefelatom enthaltenden 5- oder 6-Ring bedeuten kann;

$R_3$, $R_4$ und $R_5$, die gleich oder verschieden sein können, Wasserstoff oder einen Alkylrest mit 1 - 4 Kohlenstoffatomen bedeuten;

$R_7$ und $R_8$, die gleich oder verschieden sein können, Hydroxy; Alkoxy mit 1 - 4 Kohlenstoffatomen; oder Alkylthio mit 1 - 4 Kohlenstoffatomen bedeuten und

$R_6$ und $R_9$, die gleich oder verschieden sein können, Wasserstoff; Hydroxy; Alkoxy mit 1 - 4 Kohlenstoffatomen; Alkylthio mit 1 - 4 Kohlenstoffatomen; oder den Rest

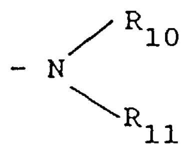

bedeuten, in dem

$R_{10}$ Wasserstoff; oder Alkyl mit 1 - 4 Kohlenstoffatomen ist und
$R_{11}$ Wasserstoff; oder Alkyl mit 1 - 4 Kohlenstoffatomen, wobei der Alkylrest gegebenenfalls durch Hydroxy, Methoxy oder Furfuryl substituiert sein kann;

sowie deren physiologisch verträglichen Salzen mit Säuren oder Komplexbildnern zur Herstellung von Mitteln zur Behandlung chronisch inflammatorischer Prozesse, der Colitis Ulcerosa und des Morbus Crohn, und von Mitteln mit antiproliferativer Wirkung.

**3.** Verwendung einer Verbindung nach Anspruch 2, worin $-NR_1R_2$ für

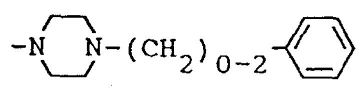

steht,
worin die Phenylgruppe durch eine oder zwei Methoxygruppen substituiert sein kann.

**4.** Verwendung einer Verbindung nach einem der Ansprüche 1 bis 3, worin $-NR_1R_2$ die Gruppe

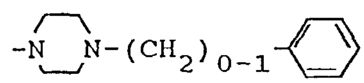

ist, worin die Phenylgruppe wie in Anspruch 1, 2 oder 3 substituiert sein kann.

**5.** Verwendung einer Verbindung nach Anspruch 1 oder 2, worin $-NR_1R_2$ die Gruppe

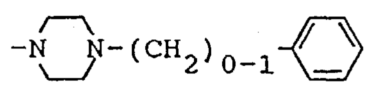

ist, worin der Phenylring ein- oder zweifach durch Fluor, Chlor, $CF_3$, Methoxy, Methyl, Ethyl oder CN substiutiert ist.

**6.** Verwendung einer Verbindung nach Anspruch 1 oder 2, worin $R_1$ Wasserstoff ist und $R_2$ ein geradkettiger oder verzweigter Alky-lrest mit 1 bis 4 Kohlenstoffatomen ist, der durch Cycloalkyl mit 3 bis 7 Kohlenstoffatomen, Thienyl oder 1 oder 2 unsubstituierte Phenylgruppen oder durch eine substituierte Phenylgruppe, deren Substituent(en) wie in Anspruch 1 oder 2 definiert sind, substituiert ist.

**7.** Verwendung einer Verbindung nach Anspruch 6, worin

$R_1$ Wasserstoff ist und $R_2$ $(C_1\text{-}C_4)$Alkylcyclohexyl oder
$R_1$ Wasserstoff ist und $R_2$ $(C_1\text{-}C_4)$Alkylphenyl, worin die Phenylgruppe unsubstituiert ist oder ein- oder zweifach substituiert ist durch F, Cl, $CF_3$, Methyl, Ethyl, Methoxy oder Ethoxy.

**8.** Verwendung einer Verbindung nach Anspruch 7, worin $R_1$ Wasserstoffist und $R_2$ - $CH_2C_6H_{11}$

**9.** Verwendung einer Verbindung nach Anspruch 1 oder 2, worin $NR_1R_2$ eine der folgenden Gruppen ist:

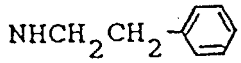

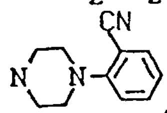

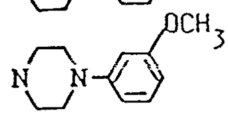

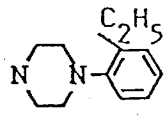

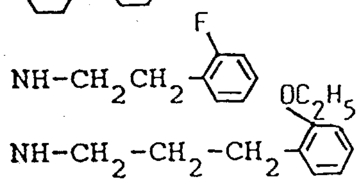

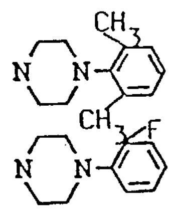

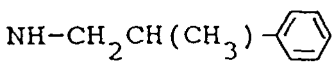

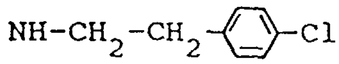

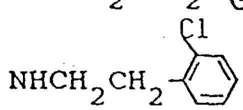

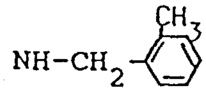

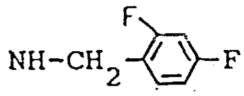

$NH-CH_2-CH(C_6H_5)_2$

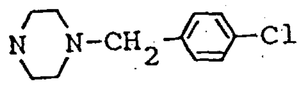

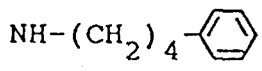

$NH-CH_2-C_6H_5$

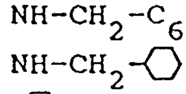

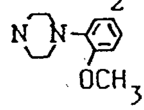

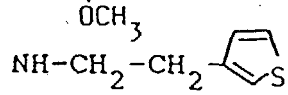

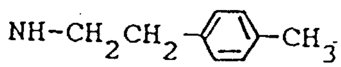

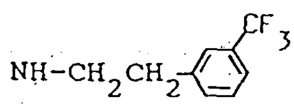

$NH-CH_2-CH_2-CH(C_6H_5)_2$ .

**10.** Verwendung einer Verbindung nach Anspruch 9, worin $NR_1R_2$ eine der folgenden Gruppen ist:

$NH-CH_2-C_6H_5$

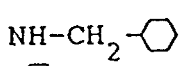

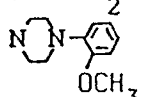

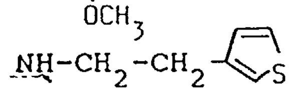

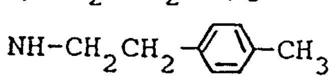

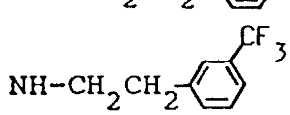

$NH-CH_2-CH_2-CH(C_6H_5)_2$ .

**11.** Verwendung einer Verbindung nach einem der Ansprüche 1 bis 10, worin $R_3$, $R_4$, $R_5$, $R_6$ und $R_9$ Wasserstoff sind und $R_7$ und $R_8$ Alkoxy mit 1-4 Kohlenstoffatomen oder $R_7$ und $R_8$ zusammen $-OCH_2O-$ oder $-OCH_2CH_2O-$ sind.

**12.** Verwendung einer Verbindung nach Anspruch 11, worin $R_7$ und $R_8$ Methoxy sind.

**13.** Verbindung der allgemeinen Formel I,

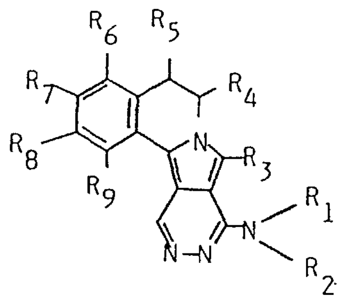

I

worin $R_3$, $R_4$, $R_5$, $R_6$ und $R_9$ Wasserstoff sind, $R_7$ und $R_8$ Alkoxy mit 1-4 Kohlenstoffatomen sind oder $R_7$ und $R_8$ zusammen $-OCH_2O-$ oder $-OCH_2CH_2O-$ sind, und die Gruppe $NR_1R_2$

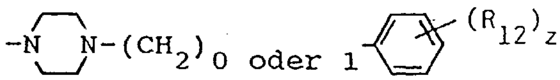

ist, worin z null, 1 oder 2 ist und $R_{12}$ CN, $CF_3$, Halogen, $(C_1-C_4)$Alkyl oder $(C_1-C_4)$Alkoxy ist, oder deren physiologisch verträgliche Salze mit Säuren oder Komplexbindnern ausgenommen die Verbindung

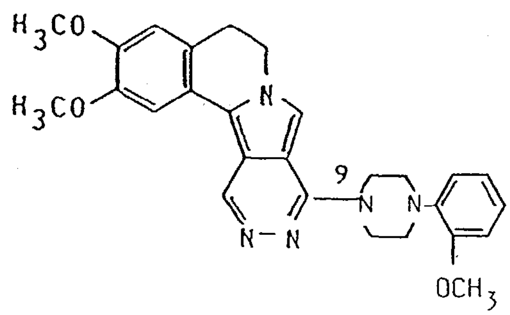

.

**14.** Verbindung nach Anspruch 13, worin $R_7$ und $R_8$ Methoxy sind.

**15.** Verbindung nach Anspruch 13 oder 14, worin $R_{12}$ CN, $OCH_3$, $CH_3$, $C_2H_5$, $C(CH_3)_3$, F, Cl oder $CF_3$ ist.

**16.** Verbindung nach einem der Ansprüche 13 bis 15, worin $-NR_1R_2$ die Gruppe

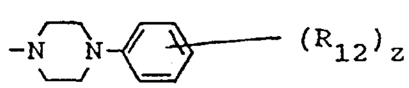

ist, worin $R_{12}$ und z wie in einem der Ansprüche 13 bis 15 definiert sind.

**17.** Verbindung nach Anspruch 13, worin $R_7$ und $R_8$ Methoxy sind und $-NR_1R_2$ für eine der folgenden Gruppen steht:

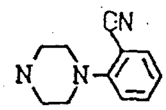

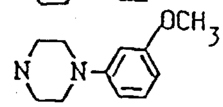

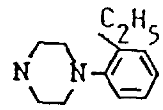

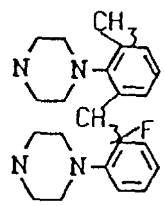

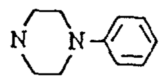

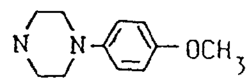

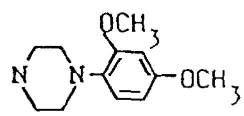

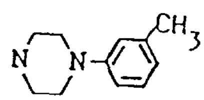

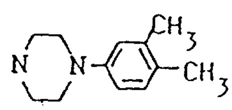

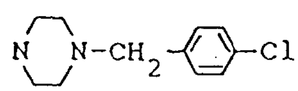

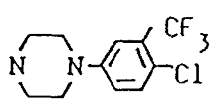

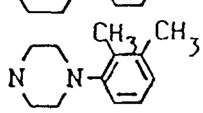

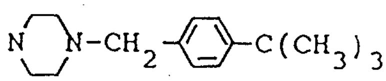

**18.** Verbindung nach Anspruch 17, worin $NR_1R_2$ für

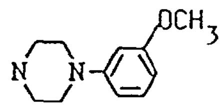

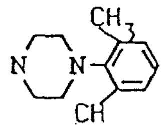

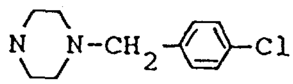

steht.

**19.** Verbindung der allgemeinen Formel I

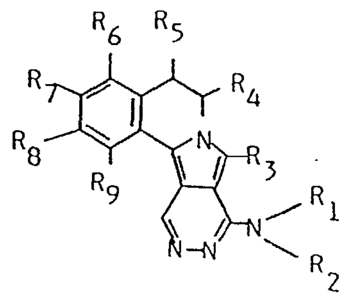

I

worin $R_3$, $R_4$, $R_5$, $R_6$ und $R_9$ Wasserstoff sind, $R_7$ und $R_8$ Alkoxy mit 1 - 4 Kohlenstoffatomen sind oder $R_7$ und $R_8$ zusammen $-OCH_2O-$ oder $-OCH_2CH_2O-$ sind und die Gruppe $-NR_1R_2$ für

$-NH(CH_2)_{1-5}-$ 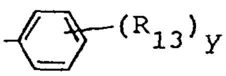

oder

$-NH(CH_2)_{1\ oder\ 2}CH(C_6H_5)_2$ steht, worin $R_{13}$ $CF_3$, $C(CH_3)_3$ oder $-OCH_2C_6H_5$ ist und y 1 oder 2 ist, oder deren physiologisch verträgliche Salze mit Säuren oder Komplexbildnern.

**20.** Verbindung nach Anspruch 19, worin $-NR_1R_2$ die Gruppe $-NH(CH_2)_{1,2}$ oder

Gruppe $-NH(CH_2)_{1,2}$ oder 3 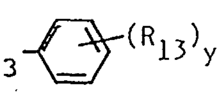

bedeutet, worin $R_{13}$ und y wie in Anspruch 19 definiert sind.

**21.** Verbindung nach einem der Ansprüche 19 bis 20, worin $R_7$ und $R_8$ Methoxy sind.

**22.** Verbindung nach Anspruch 19, worin $R_7$ und $R_8$ Methoxy sind und $-NR_1R_2$ eine der folgenden Bedeutungen hat:

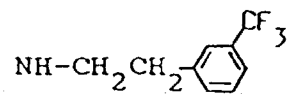

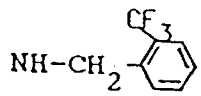

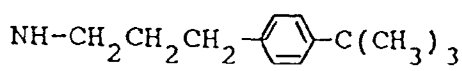

$NH\text{-}CH_2\text{-}CH_2\text{-}CH(C_6H_5)_2$

$NH\text{-}CH_2\text{-}CH(C_6H_5)_2$

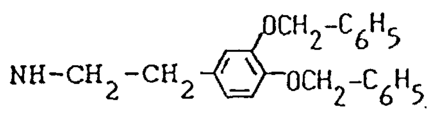

**23.** Verbindung der allgemeinen Formel I

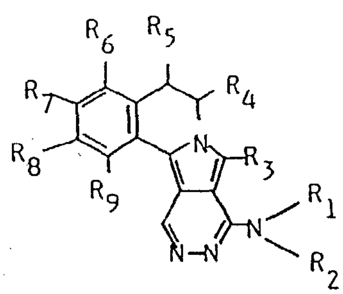

I

worin $R_3$, $R_4$, $R_5$, $R_6$ und $R_9$ Wasserstoff sind, $R_7$ und $R_8$ Methoxy sind und $-NR_1R_2$ eine der folgenden Bedeutungen hat:

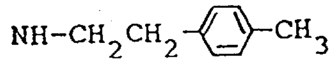

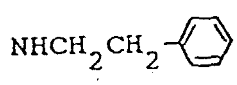

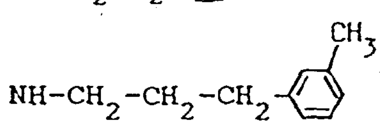

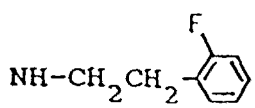

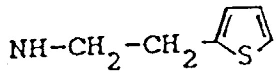

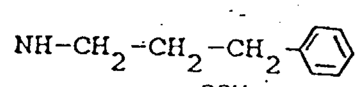

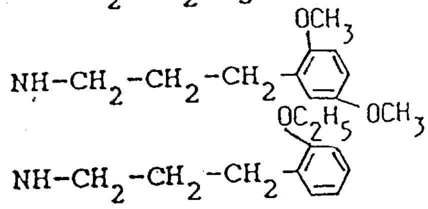

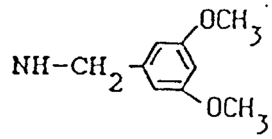

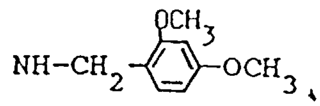

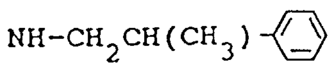

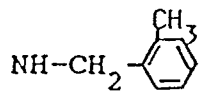

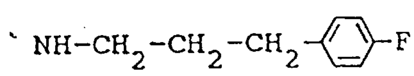

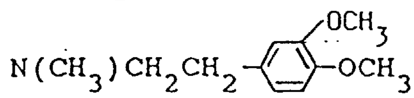

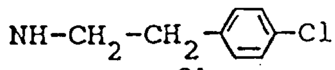

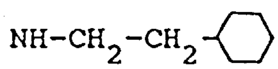

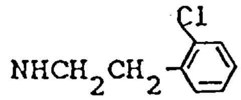

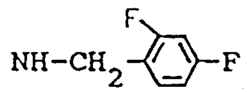

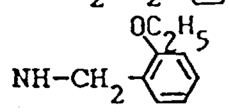

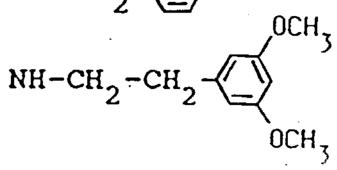

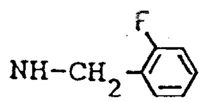

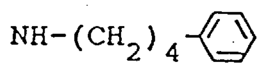

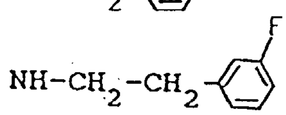

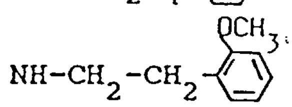

oder deren physiologisch verträgliche Salze mit Säuren oder Komplexbildnern.

**24.** Verbindung nach Anspruch 23, worin $-NR_1R_2$ eine der folgenden Bedeutungen hat:

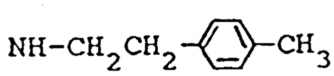

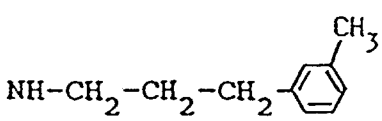

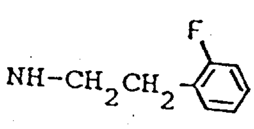

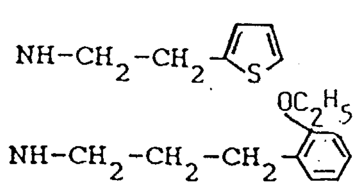

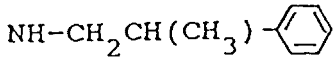

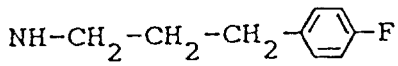

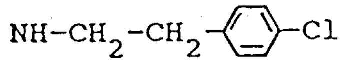

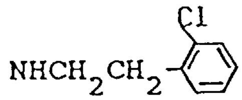

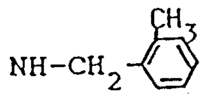

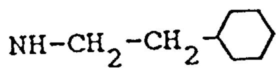

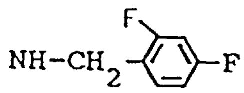

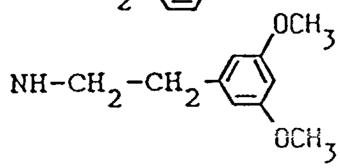

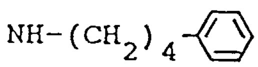 .

**25.** Verfahren zur Herstellung von Verbindungen der allgemeinen Formel I gemäß einem der Ansprüche 13 bis 24 oder deren physiologisch verträglichen Salzen mit Säuren oder Komplexbildnern, dadurch gekennzeichnet, daß man eine Verbindung der allgemeinen Formel II

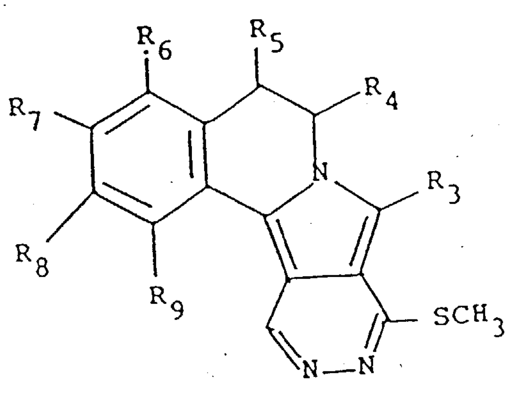

(II)

worin

die Reste $R_3$, $R_4$, $R_5$, $R_6$, $R_7$, $R_8$ und
$R_9$ die oben angegebenen Bedeutungen haben, mit einer Verbindung der allgemeinen Formel

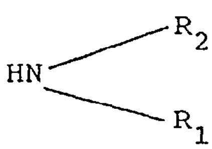

(III)

worin
$R_1$ und $R_2$ die oben angegebene Bedeutung haben, umsetzt, und daß man ein so erhaltenes Endprodukt gegebenenfalls in an sich bekannter Weise in ein physiologisch verträgliches Salz überführt.

**26.** Pharmazeutische Präparate, enthaltend als Wirkstoff eine oder mehrere Verbindungen gemäß einem der Ansprüche 13 bis 24 in Kombination mit üblichen Hilfs- oder Trägerstoffen.

**27.** Verfahren zur Herstellung pharmazeutischer Präparate nach Anspruch 26, dadurch gekennzeichnet, daß man Verbindungen gemäß einem der Ansprüche 13 bis 24 mit üblichen galenischen Hilfs- und/oder Trägerstoffen zu üblichen pharmazeutischen Anwendungsformen verarbeitet.

**Claims**

**1.** Use of compounds of formula (I)

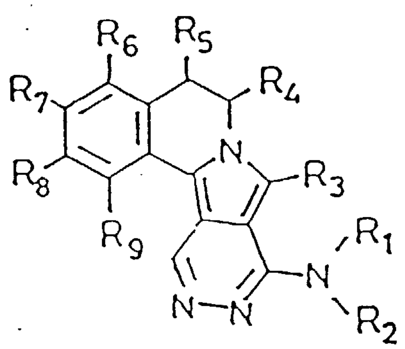

(I)

wherein

$R_1$ and $R_2$, which may be identical or different, denote hydrogen; $C_{3-7}$-cycloalkyl; $C_{2-5}$-alkenyl; phenyl (wherein the phenyl ring may optionally be mono- or disubstituted by halogen or methoxy); propargyl; a straight-chained or branched, saturated or unsaturated $C_{1-5}$-alkyl, which may be substituted by hydroxy, $C_{1-4}$-alkoxy, halogen, NH2, NH-alkyl having 1 to 2 carbon atoms, N,N-di($C_{1-2}$)alkylamino, NH-acyl having 2 to 4 carbon atoms, $C_{3-7}$-cycloalkyl, 1 or 2 phenyl groups (wherein the phenyl ring or rings may in turn be mono- or disubstituted by halogen, $CF_3$, $C_{1-4}$-alkyl, $C_{1-2}$-alkoxyl NH-alkyl having 1 to 2 carbon atoms, N,N-dialkyl having 1 to 2 carbon atoms, $NH_2$, N-acyl having 2 to 3 carbon atoms, alkylsulphonylamino or benzyloxy), furyl, thienyl, a nitrogen-containing heterocyclic 5- or 6-membered ring which may optionally contain as further heteroatom an oxygen or sulphur atom (whilst the ring may optionally be substituted by $C_{1-4}$-alkyl); or

$R_1$ and $R_2$ together with the nitrogen atom denote a 3- to 7-membered ring which may optionally contain, as a further heteroatom, an oxygen or nitrogen atom, whilst this ring is optionally substituted by phenyl-($C_{0-4}$)-alkyl (whilst the phenyl ring may in turn be mono- or disubstituted by halogen, $CF_3$, ($C_{1-4}$)alkoxy, ($C_{1-4}$)alkyl or CN, whilst the substituents may be identical or different); or

if $R_1$ denotes hydrogen, R2 may also represent -$NH_2$; di ($C_{1-2}$) alkylamino; acetonylamino; -NH ($C_{2-3}$) acyl; an alkylsulphonyl or alkoxycarbonyl group having 1 to 3 carbon atoms in the alkyl chain; the isopropylideneamino group

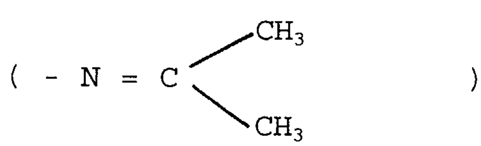

or a heterocyclic 5- or 6-membered ring containing a nitrogen atom and optionally an oxygen, nitrogen or sulphur atom as a further heteroatom;

$R_3$, $R_4$ and $R_5$, which may be identical or different denote hydrogen or a $C_{1-4}$-alkyl group;

$R_7$ and $R_8$, which may be identical or different, represent hydroxy; $C_{1-4}$-alkoxy; or $C_{1-4}$-alkylthio and

$R_6$ and $R_9$, which may be identical or different, denote hydrogen; hydroxy; $C_{1-4}$-alkoxy; $C_{1-4}$-alkythio; or the group

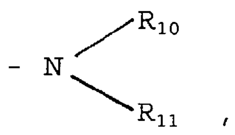

wherein

$R_{10}$ denotes hydrogen; or $C_{1-4}$-alkyl and
$R_{11}$ denotes hydrogen; or $C_{1-4}$-alkyl, whilst the alkyl group may optionally be substituted by hydroxy, methoxy or furfuryl;

or 2 adjacent substituents of the substituents $R_6$, $R_7$, $R_8$ and $R_9$ together form the group $-O-(CH_2)_{1 \text{ or } 2}-O-$ and the other two substituents are as hereinbefore defined;

and the physiologically acceptable salts thereof with acids or complexing agents for preparing agents for treating chronic inflammatory processes, ulcerative colitis and Crohn's disease, and for preparing agents having an antiproliferative activity.

**2.** Use of a compound of formula (I) according to claim 1, wherein

$R_1$ and $R_2$, which may be identical or different, represent hydrogen; $C_{3-7}$-cycloalkyl; $C_{2-5}$-alkenyl; phenyl (wherein the phenyl ring is optionally mono- or disubstituted by halogen or methoxy); propargyl; a straight-chained or branched, saturated or unsaturated $C_{1-5}$-alkyl group which may be substituted by hydroxy, $C_{1-4}$-alkoxy, halogen, $NH_2$, NH-alkyl having 1 to 2 carbon atoms, N,N-di($C_{1-2}$)alkylamino, NH-acyl having 2 to 4 carbon atoms, $C_{3-7}$-cycloalkyl, phenyl (whilst the phenyl ring may in turn be mono- or disubstituted by halogen, $C_{1-2}$-alkyl, $C_{1-2}$-alkoxy, NH-alkyl having 1 to 2 carbon atoms, N,N-dialkyl having 1 to 2 carbon atoms, $NH_2$, N-acyl having 2 to 3 carbon atoms or alkylsulphonylamino), furyl, thienyl, a nitrogen-containing heterocyclic 5- or 6-membered ring which may optionally contain an oxygen or sulphur atom as a further heteroatom (whilst the ring is optionally substituted by $C_{1-4}$-alkyl); or

$R_1$ and $R_2$ together with the nitrogen atom denote a 3- to 7-membered ring which may optionally contain an oxygen or nitrogen atom as a further heteroatom, whilst this ring is optionally substituted by phenyl-($C_{0-4}$)alkyl (whilst the phenyl ring is in turn mono- or disubstituted by halogen or methoxy); or

$R_2$, if $R_1$ denotes hydrogen, may also denote $-NH_2$; di($C_{1-2}$)alkylamino; acetonylamino; $-NH(C_{2-3})$acyl; an alkylsulphonyl or alkoxycarbonyl group each having 1 to 3 carbon atoms in the alkyl chain; the isopropylideneamino group

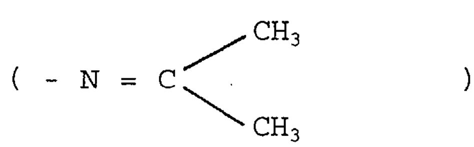

or a heterocyclic 5- or 6-membered ring containing a nitrogen atom and optionally, as a further heteroatom, an oxygen, nitrogen or sulphur atom;

$R_3$, $R_4$ and $R_5$, which may be identical or different, denote hydrogen or a $C_{1-4}$-alkyl group;

$R_7$ and $R_8$, which may be identical or different, denote hydroxy; $C_{1-4}$-alkoxy; or $C_{1-4}$-alkylthio and

$R_6$ and $R_9$, which may be identical or different, denote hydrogen; hydroxy; $C_{1-4}$-alkoxy; $C_{1-4}$-alkylthio; or the group

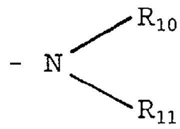 ,

wherein

$R_{10}$ denotes hydrogen; or $C_{1-4}$-alkyl and
$R_{11}$ denotes hydrogen; or $C_{1-4}$-alkyl, whilst the alkyl group may optionally be substituted by hydroxy, methoxy or furfuryl;

and the physiologically acceptable salts thereof with acids or complexing agents for preparing agents for treating chronic inflammatory processes, ulcerative colitis and Crohn's disease, and for producing agents with an antiproliferative activity.

**3.** Use of a compound according to claim 2 wherein $-NR_1R_2$ represents

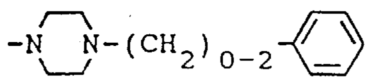 ,

wherein the phenyl group may be substituted by one or two methoxy groups.

**4.** Use of a compound according to one of claims 1 to 3, wherein
$-NR_1R_2$ denotes the group

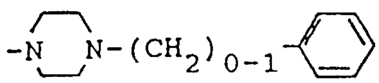

wherein the phenyl group may be substituted as in any of claims 1, 2 or 3..

**5.** Use of a compound according to claim 1 or 2, wherein $-NR_1R_2$ denotes the group

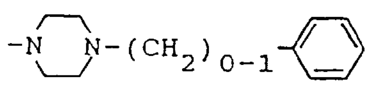

wherein the phenyl ring is mono- or disubstituted by fluorine, chlorine, $CF_3$, methoxy, methyl, ethyl or CN.

**6.** Use of a compound according to claim 1 or 2, wherein $R_1$ is hydrogen and $R_2$ is a straight-chained or branched $C_{1-4}$-alkyl group which is substituted by $C_{3-7}$-cycloalkyl, thienyl or 1 or 2 unsubstituted phenyl groups or a substituted phenyl group the substituent(s) of which is or are defined as in claim 1 or 2.

**7.** Use of a compound according to claim 6, wherein $R_1$ is hydrogen and $R_2$ is $(C_{1-4})$alkylcyclohexyl or $R_1$ is hydrogen and $R_2$ is $(C_{1-4})$alkylphenyl, wherein the phenyl group is unsubstituted or mono- or disubstituted by F, Cl, $CF_3$, methyl, ethyl, methoxy or ethoxy.

**8.** Use of a compound according to claim 7, wherein $R_1$ is hydrogen and $R_2$ is $-CH_2C_6H_{11}$.

**9.** Use of a compound according to claim 1 or 2, wherein $NR_1R_2$ is one of the following groups:

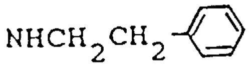

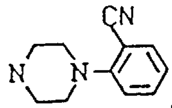

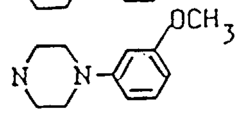

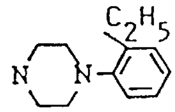

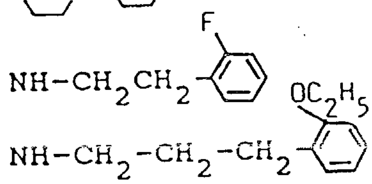

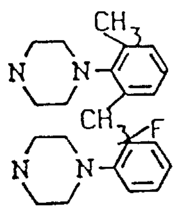

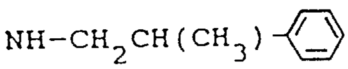

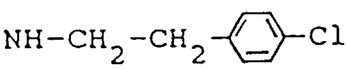

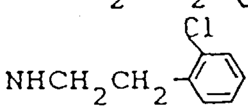

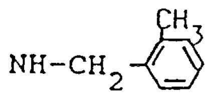

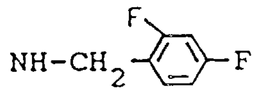

$NH-CH_2-CH(C_6H_5)_2$

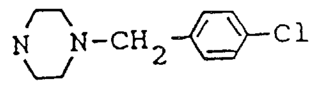

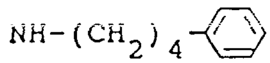

$NH-CH_2-C_6H_5$

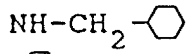

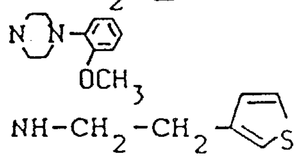

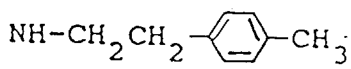

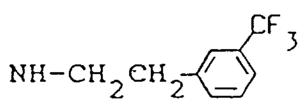

$NH-CH_2-CH_2-CH(C_6H_5)_2$

**10.** Use of a compound according to claim 9 wherein $NR_1R_2$ is one of the following groups:

$NH-CH_2-C_6H_5$

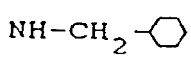

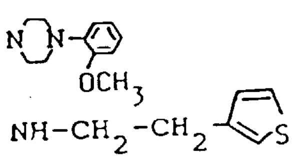

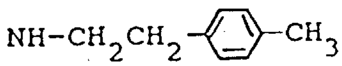

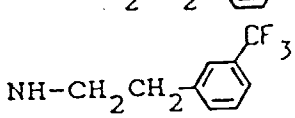

$NH\text{-}CH_2\text{-}CH_2\text{-}CH(C_6H_5)_2$ .

**11.** Use of a compound according to one of claims 1 to 10, wherein $R_3$, $R_4$, $R_5$, $R_6$ and $R_9$ denote hydrogen and $R_7$ and $R_8$ represent $C_{1\text{-}4}$-alkoxy or $R_7$ and $R_8$ together represent $-OCH_2O-$ or $-OCH_2CH_2O-$.

**12.** Use of a compound according to claim 11 wherein $R_7$ and $R_8$ are methoxy.

**13.** Compounds of general formula I

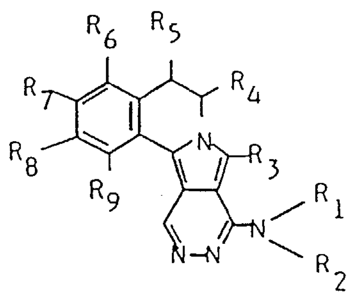

(I)

wherein $R_3$, $R_4$, $R_5$, $R_6$ and $R_9$ denote hydrogen, $R_7$ and $R_8$ denote $C_{1\text{-}4}$-alkoxy or $R_7$ and $R_8$ together represent $-OCH_2O-$ or $-OCH_2CH_2O-$, and the group $NR_1R_2$ represents

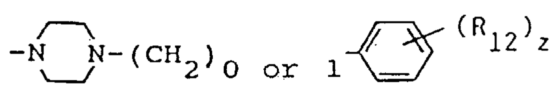

wherein z is zero, 1 or 2 and $R_{12}$ is CN, $CF_3$, halogen, $(C_{1\text{-}4})$alkyl or $(C_{1\text{-}4})$alkoxy, or the physiologically acceptable salts thereof with acids or complexing agents, with the exception of the compound

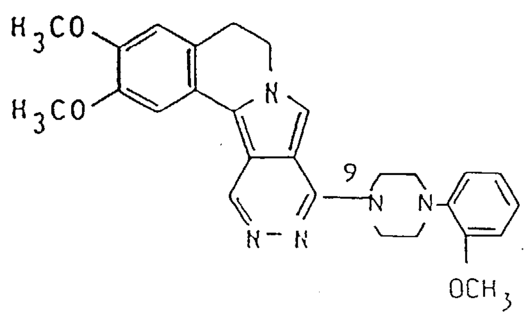

**14.** Compound according to claim 13, wherein $R_7$ and $R_8$ represent methoxy.

**15.** Compound according to claim 13 or 14, wherein $R_{12}$ is CN, $OCH_3$, $CH_3$, $C_2H_5$, $C(CH_3)_3$, F, Cl or $CF_3$.

**16.** Compound according to one of claims 13 to 15, wherein $-NR_1R_2$ is the group

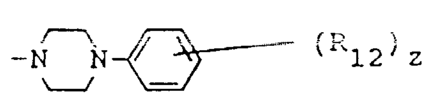

wherein $R_{12}$ and z are defined as in one of claims 13 to 15.

**17.** Compounds according to claim 13, wherein $R_7$ and $R_8$ are methoxy and $-NR_1R_2$ represents one of the following groups:

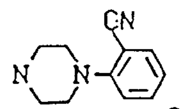

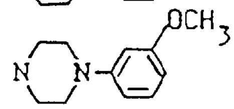

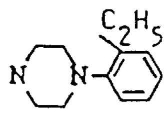

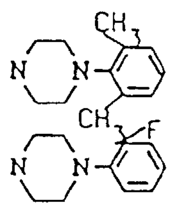

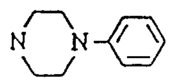

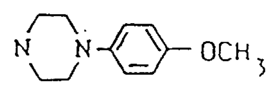

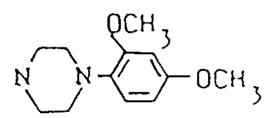

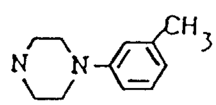

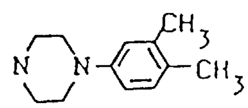

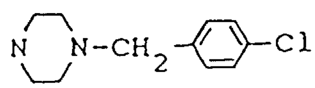

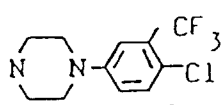

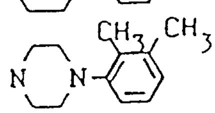

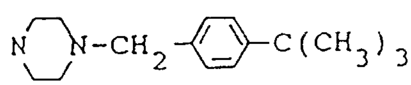

**18.** Compounds according to claim 17 wherein $NR_1R_2$ represents

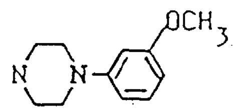

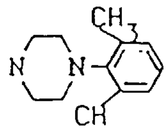

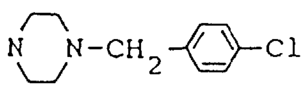

**19.** Compounds of general formula I

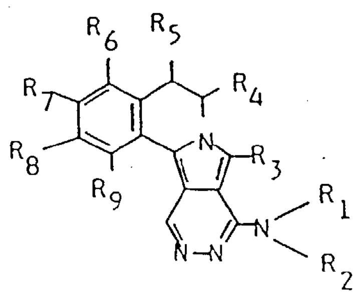

I

wherein $R_3$, $R_4$, $R_5$, $R_6$ and $R_9$ denote hydrogen, $R_7$ and $R_8$ denote $C_{1-4}$-alkoxy or $R_7$ and $R_8$ together denote $-OCH_2O-$ or $-OCH_2CH_2O-$ and the group $-NR_1R_2$ denotes

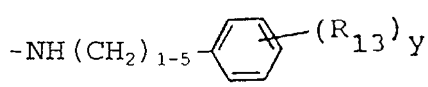

or

$-NH(CH_2)_{1\ \text{or}\ 2}CH(C_6H_5)_2$, wherein $R_{13}$ is $CF_3$, $C(CH_3)_3$ or $-OCH_2C_6H_5$ and y represents 1 or 2, or the physiologically acceptable salts thereof with acids or complexing agents.

**20.** Compounds according to claim 19, wherein $-NR_1R_2$ represents the group

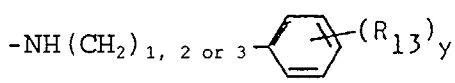

wherein $R_{13}$ and y are defined as in claim 19.

**21.** Compound according to one of claims 19 to 20, wherein $R_7$ and $R_8$ represent methoxy.

**22.** Compound according to claim 19, wherein $R_7$ and $R_8$ denote methoxy and $-NR_1R_2$ has one of the following meanings:

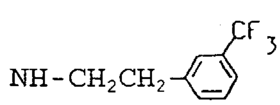

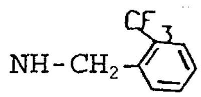

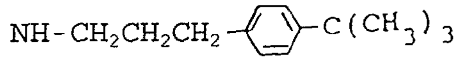

NH-$CH_2$-$CH_2$-$CH(C_6H_5)_2$

NH-$CH_2$-$CH(C_6H_5)_2$

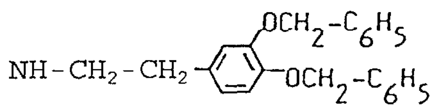

**23.** Compound of general formula I

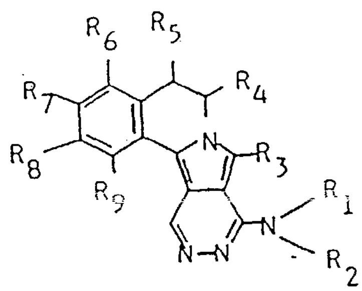

I

wherein $R_3$, $R_4$, $R_5$, $R_6$ and $R_9$ represent hydrogen, $R_7$ and $R_8$ represent methoxy and $-NR_1R_2$ has one of the following meanings:

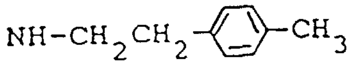

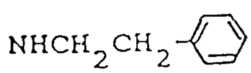

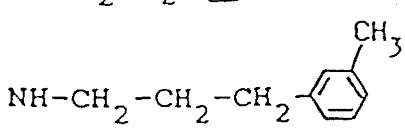

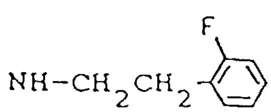

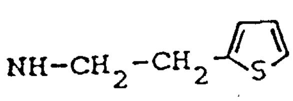

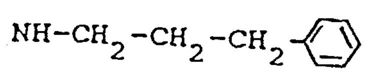

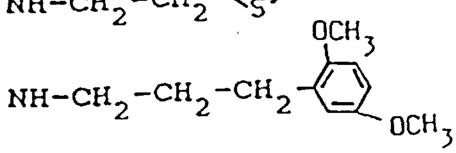

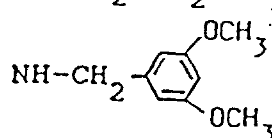

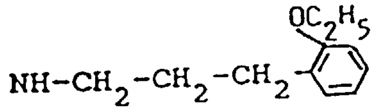
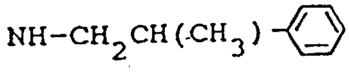
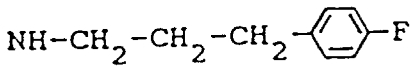
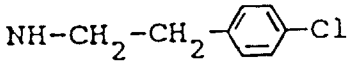
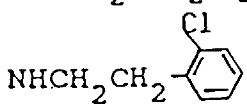
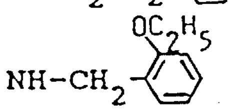
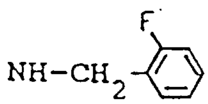
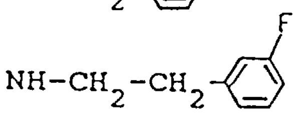
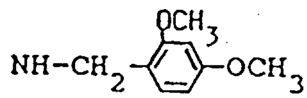
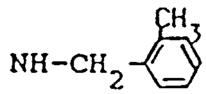
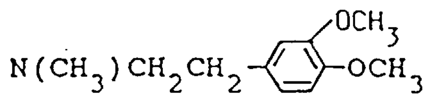
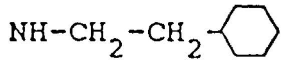
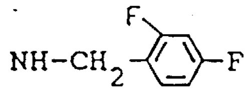
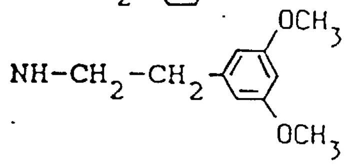
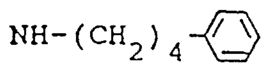
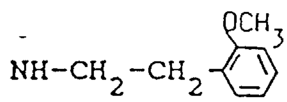

or the physiologically acceptable salts thereof with acids or complexing agents.

**24.** Compound according to claim 23, wherein $-NR_1R_2$ has one of the following meanings:

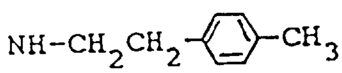
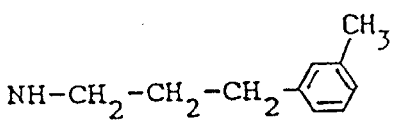
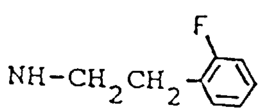
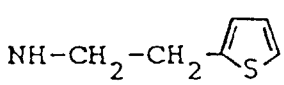
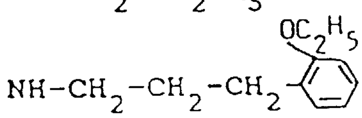
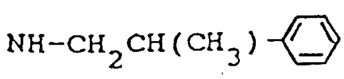
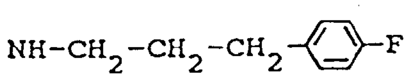

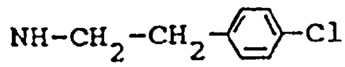

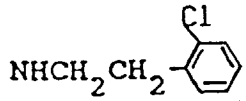

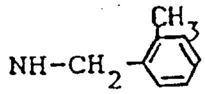

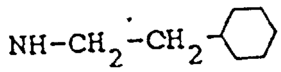

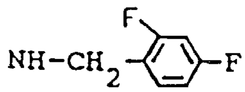

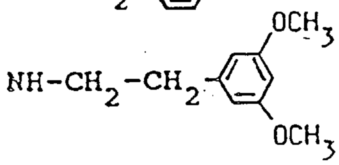

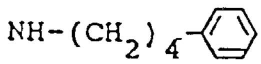

**25.** Process for preparing compounds of general formula I according to one of claims 13 to 24 or physiologically acceptable salts thereof with acids or complexing agents, characterised in that a compound of general formula II

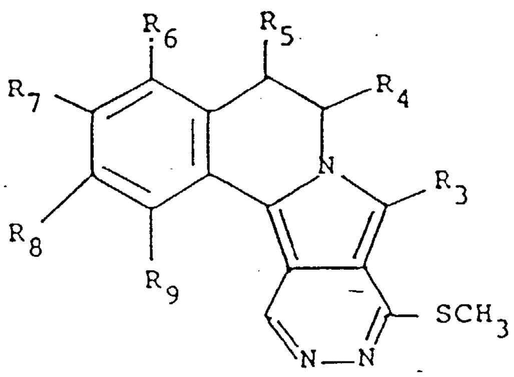

(II)

wherein the groups $R_3$, $R_4$, $R_5$, $R_6$, $R_7$, $R_8$ and $R_9$ are as hereinbefore defined, is reacted with a compound of general formula

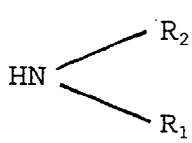

(III)

wherein
$R_1$ and $R_2$ are as hereinbefore defined, and an end product thus obtained is optionally converted into a physiologically acceptable salt in a manner known per se.

**26.** Pharmaceutical preparations containing as active substance one or more compounds according to any one of claims 13 to 24 in conjunction with conventional excipients or carriers.

**27.** Process for producing pharmaceutical preparations according to claim 26, characterised in that compounds according to any one of claims 13 to 24 are processed with conventional galenic excipients and/or carriers to form pharmaceutical preparations.

## Revendications

1. Utilisation de composés de formule (I)

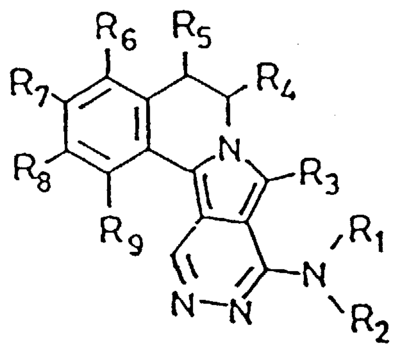

(I)

dans laquelle

$R_1$ et $R_2$, qui peuvent être identiques ou différents, représentent l'hydrogène, cycloalkyle en $C_3$-$C_7$, alcényle en $C_2$-$C_5$, phényle (le cycle phényle étant éventuellement substitué une ou deux fois par halogène ou méthoxy), propargyle, un reste alkyle linéaire ou ramifié, saturé ou insaturé, de 1 à 5 atomes de carbone, qui peut être substitué par hydroxyle, alcoxy de 1 à 4 atomes de carbone, halogène, $NH_2$, NH-alkyle de 1 à 2 atomes de carbone, N,N-di(alkyle en $C_1$-$C_2$)amino, NH-acyle de 2 à 4 atomes de carbone, cycloalkyle de 3 à 7 atomes de carbone, 1 ou 2 groupes phényle (le/les cycles phényle pouvant être substitués eux-mêmes une ou deux fois par halogène, $CF_3$, alkyle de 1 à 4 atomes de carbone, alcoxy de 1 à 2 atomes de carbone, NH-alkyle de 1 à 2 atomes de carbone, N,N-dialkyle de 1 à 2 atomes de carbone, $NH_2$, N-acyle de 2 à 3 atomes de carbone, alkylsulfonylamino ou benzyloxy), furyle, thiényle, un cycle hétérocyclique azoté à 5 ou 6 chaînons qui peut éventuellement contenir comme autre hétéroatome un atome d'oxygène ou de soufre (le cycle étant éventuellement substitué par alkyle de 1 à 4 atomes de carbone), ou
$R_1$ et $R_2$ forment avec l'atome d'azote un cycle à 3 à 7 chaînons qui peut éventuellement contenir comme autre hétéroatome un atome d'oxygène ou d'azote, ce cycle étant éventuellement substitué par phényl-(alkyle en $C_0$-$C_4$) (le cycle phényle pouvant pour sa part être substitué une ou deux fois par halogène, $CF_3$, alcoxy en $C_1$-$C_4$, alkyle en $C_1$-$C_4$ ou CN, les substituants pouvant être identiques ou différents), ou
$R_2$, au cas où $R_1$ représente l'hydrogène, peut aussi représenter $-NH_2$, di(alkyle en $C_1$-$C_2$)amino, acétonylamino, -NH-acyle en $C_2$-$C_3$, un reste alkylsulfonyle ou alcoxycarbonyle ayant dans chaque cas 1 à 3 atomes de carbone dans la chaîne alkyle, le groupe isopropylidèneamino

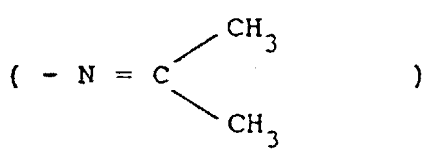

ou un cycle hétérocyclique à 5 ou 6 chaînons contenant un atome d'azote et éventuellement comme autre hétéroatome un atome d'oxygène, d'azote ou de soufre ;
$R_3$, $R_4$ et $R_5$, qui peuvent être identiques ou différents, représentent l'hydrogène ou un reste alkyle de 1 à 4 atomes de carbone ;
$R_7$ et $R_8$, qui peuvent être identiques ou différents, représentent hydroxyle, alcoxy de 1 à 4 atomes de carbone ou alkylthio de 1 à 4 atomes de carbone et
$R_6$ et $R_9$, qui peuvent être identiques ou différents, représentent l'hydrogène, hydroxyle, alcoxy de 1 à 4 atomes de carbone, alkylthio de 1 à 4 atomes de carbone, ou le reste

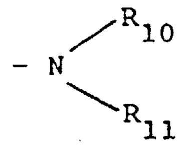

dans lequel

$R_{10}$ représente l'hydrogène ou alkyle de 1 à 4 atomes de carbone et $R_{11}$ représente l'hydrogène ou alkyle de 1 à 4 atomes de carbone, le reste alkyle pouvant éventuellement être substitué par hydroxyle, méthoxy ou furfuryle ;

ou 2 substituants voisins des substituants $R_6$, $R_7$, $R_8$ et $R_9$ forment ensemble le groupe $-O-(CH_2)_{1 \text{ ou } 2}-O-$ et les 2 substituants restants dans chaque cas sont définis comme ci-dessus ;

ainsi que de leurs sels physiologiquement acceptables avec des acides ou des complexants pour la préparation d'agents pour le traitement des processus inflammatoires chroniques, de la colite ulcéreuse et de la maladie de Crohn, et d'agents à effet antiprolifératif.

**2.** Utilisation d'un composé de formule (I) selon la revendication 1, dans lequel

$R_1$ et $R_2$, qui peuvent être identiques ou différents, représentent l'hydrogène, cycloalkyle en $C_3$-$C_7$, alcényle en $C_2$-$C_5$, phényle (le cycle phényle étant éventuellement substitué une ou deux fois par halogène ou méthoxy), propargyle, un reste alkyle linéaire ou ramifié, saturé ou insaturé, de 1 à 5 atomes de carbone, qui peut être substitué par hydroxyle, alcoxy de 1 à 4 atomes de carbone, halogène, $NH_2$, NH-alkyle de 1 à 2 atomes de carbone, N,N-di(alkyle en $C_1$-$C_2$)amino, NH-acyle de 2 à 4 atomes de carbone, cycloalkyle de 3 à 7 atomes de carbone, phényle (le cycle phényle pouvant lui-même être substitué une ou deux fois par halogène, alkyle de 1 à 2 atomes de carbone, alcoxy de 1 à 2 atomes de carbone, NH-alkyle de 1 à 2 atomes de carbone, N, N-dialkyle de 1 à 2 atomes de carbone, $NH_2$, N-acyle de 2 à 3 atomes de carbone ou alkylsulfonylamino), furyle, thiényle, un cycle hétérocyclique azoté à 5 ou 6 chaînons qui peut éventuellement contenir comme autre hétéroatome un atome d'oxygène ou de soufre (le cycle étant éventuellement substitué par alkyle de 1 à 4 atomes de carbone), ou

$R_1$ et $R_2$ forment avec l'atome d'azote un cycle à 3 à 7 chaînons qui peut éventuellement contenir comme autre hétéroatome un atome d'oxygène ou d'azote, ce cycle étant éventuellement substitué par phényl-(alkyle en $C_0$-$C_4$) (le cycle phényle pouvant pour sa part être substitué une ou deux fois par halogène ou méthoxy), ou

$R_2$, au cas où $R_1$ représente l'hydrogène, peut aussi représenter $-NH_2$, di(alkyle en $C_1$-$C_2$)amino, acétonylamino, -NH-acyle en $C_2$-$C_3$, un reste alkylsulfonyle ou alcoxycarbonyle ayant dans chaque cas 1 à 3 atomes de carbone dans la chaîne alkyle, le groupe isopropylidèneamino

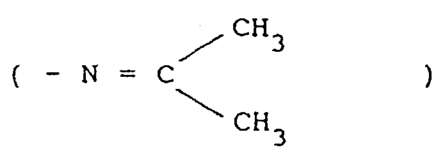

ou un cycle hétérocyclique à 5 ou 6 chaînons contenant un atome d'azote et éventuellement comme autre hétéroatome un atome d'oxygène, d'azote ou de soufre ;

$R_3$, $R_4$ et $R_5$, qui peuvent être identiques ou différents, représentent l'hydrogène ou un reste alkyle de 1 à 4 atomes de carbone ;

$R_7$ et $R_8$, qui peuvent être identiques ou différents, représentent hydroxyle, alcoxy de 1 à 4 atomes de carbone ou alkylthio de 1 à 4 atomes de carbone et

$R_6$ et $R_9$, qui peuvent être identiques ou différents, représentent l'hydrogène, hydroxyle, alcoxy de 1 à 4 atomes de carbone, alkylthio de 1 à 4 atomes de carbone, ou le reste

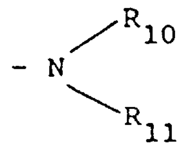

dans lequel

$R_{10}$ représente l'hydrogène ou alkyle de 1 à 4 atomes de carbone et $R_{11}$ représente l'hydrogène ou alkyle de 1 à 4 atomes de carbone, le reste alkyle pouvant éventuellement être substitué par hydroxyle, méthoxy ou furfuryle ;
ainsi que de ses sels physiologiquement acceptables avec des acides ou des complexants pour la préparation d'agents pour le traitement des processus inflammatoires chroniques, de la colite ulcéreuse et de la maladie de Crohn, et d'agents à effet antiprolifératif.

**3.** Utilisation d'un composé selon la revendication 2 dans lequel $-NR_1R_2$ représente

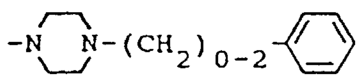

où le groupe phényle peut être substitué par un ou deux groupes méthoxy.

**4.** Utilisation d'un composé selon l'une des revendications 1 à 3 dans lequel $-NR_1R_2$ est le groupe

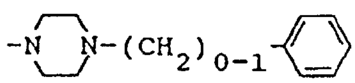

où le groupe phényle peut être substitué comme dans la revendication 1, 2 ou 3.

**5.** Utilisation d'un composé selon la revendication 1 ou 2 dans lequel $-NR_1R_2$ est le groupe

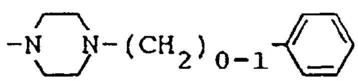

où le cycle phényle est substitué une ou deux fois par fluor, chlore, $CF_3$, méthoxy, méthyle, éthyle ou CN.

**6.** Utilisation d'un composé selon la revendication 1 ou 2 dans lequel $R_1$ est l'hydrogène et $R_2$ est un reste alkyle linéaire ou ramifié de 1 à 4 atomes de carbone qui est substitué par cycloalkyle de 3 à 7 atomes de carbone, thiényle ou 1 ou 2 groupes phényle non substitués ou par un groupe phényle substitué dont le ou les substituants sont définis comme dans la revendication 1 ou 2.

**7.** Utilisation d'un composé selon la revendication 6 dans lequel $R_1$ est l'hydrogène et $R_2$ est (alkyle en $C_1$-$C_4$)cyclohexyle ou $R_1$ est l'hydrogène et $R_2$ est (alkyle en $C_1$-$C_4$)phényle, le groupe phényle étant non substitué ou substitué une ou deux fois par F, Cl, $CF_3$, méthyle, éthyle, méthoxy ou éthoxy.

**8.** Utilisation d'un composé selon la revendication 7 dans lequel $R_1$ est l'hydrogène et $R_2$ est $-CH_2C_6H_{11}$.

**9.** Utilisation d'un composé selon la revendication 1 ou 2 dans lequel $NR_1R_2$ est l'un des groupes suivants :

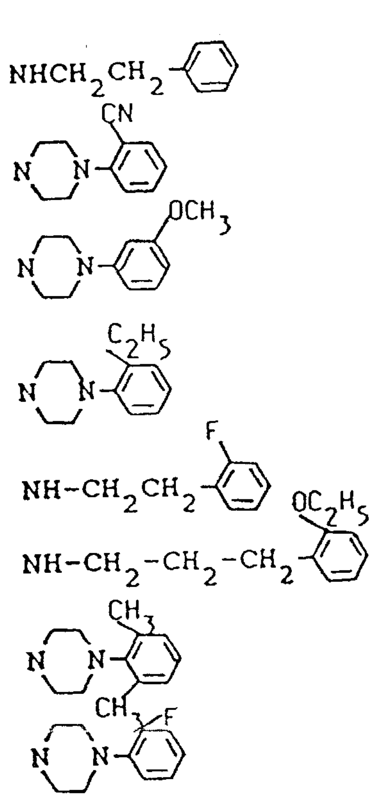

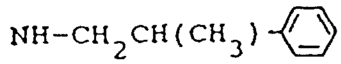

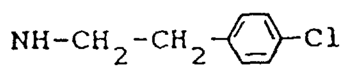

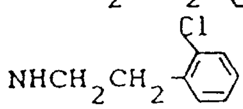

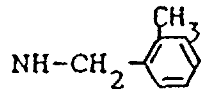

$NH-CH_2-CH(C_6H_5)_2$

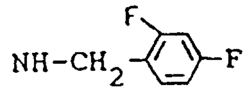

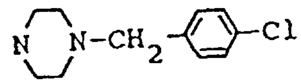

$NH-CH_2-C_6H_5$

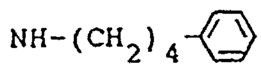

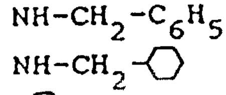

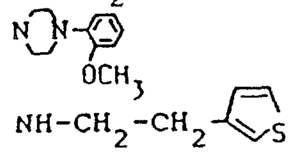

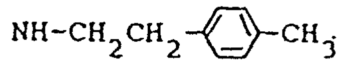

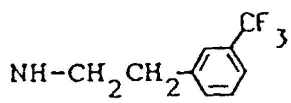

$NH-CH_2-CH_2-CH(C_6H_5)_2$ .

**10.** Utilisation d'un composé selon la revendication 9 dans lequel $NR_1R_2$ est l'un des groupes suivants :

$NH-CH_2-C_6H_5$

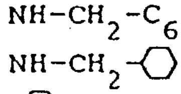

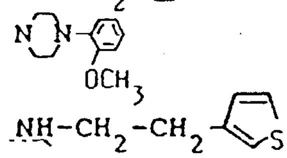

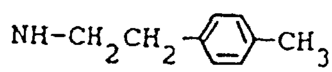

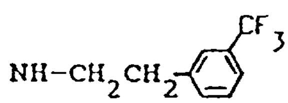

$NH-CH_2-CH_2-CH(C_6H_5)_2$ .

**11.** Utilisation d'un composé selon l'une des revendications 1 à 10 dans lequel $R_3$, $R_4$, $R_5$, $R_6$ et $R_9$ sont l'hydrogène et $R_7$ et $R_8$ sont alcoxy de 1 à 4 atomes de carbone ou $R_7$ et $R_8$ sont ensemble $-OCH_2O-$ ou $-OCH_2CH_2O-$.

**12.** Utilisation d'un composé selon la revendication 11 dans lequel $R_7$ et $R_8$ sont méthoxy.

**13.** Composé de formule générale I

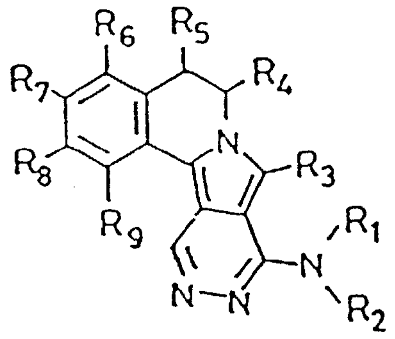

(I)

où $R_3$, $R_4$, $R_5$, $R_6$ et $R_9$ sont l'hydrogène, $R_7$ et $R_8$ sont alcoxy de 1 à 4 atomes de carbone ou $R_7$ et $R_8$ sont ensemble $-OCH_2O-$ ou $-OCH_2CH_2O-$, et le groupe $NR_1R_2$ est

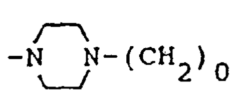 ou 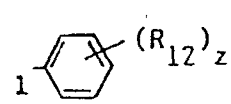

où z est 0, 1 ou 2 et $R_{12}$ est CN, $CF_3$, halogène, alkyle en $C_1$-$C_4$ ou alcoxy en $C_1$-$C_4$, ou ses sels physiologiquement acceptables avec des acides ou des complexants à l'exception du composé

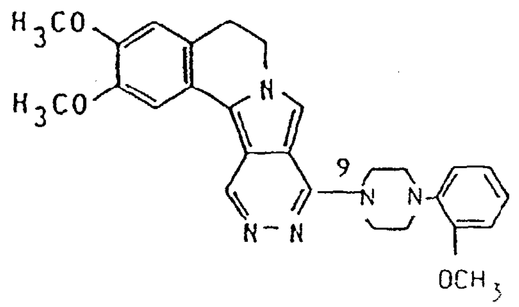

**14.** Composé selon la revendication 13 dans lequel $R_7$ et $R_8$ sont méthoxy.

**15.** Composé selon la revendication 13 ou 14 dans lequel $R_{12}$ est CN, $OCH_3$, $CH_3$, $C_2H_5$, $C(CH_3)_3$, F, Cl ou $CF_3$.

**16.** Composé selon l'une des revendications 13 à 15 dans lequel $-NR_1R_2$ est le groupe

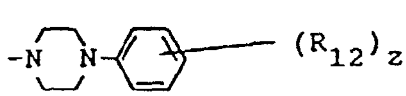

où $R_{12}$ et z sont définis comme dans l'une des revendications 13 à 15.

**17.** Composé selon la revendication 13 dans lequel $R_7$ et $R_8$ sont méthoxy et $-NR_1R_2$ représente l'un des groupes suivants :

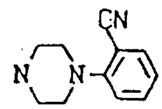

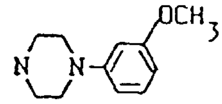

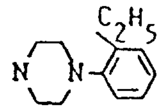

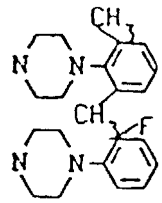

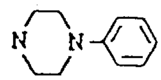

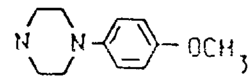

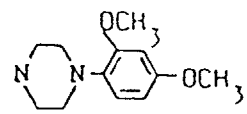

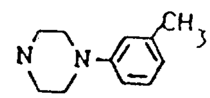

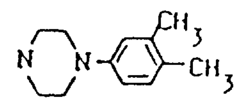

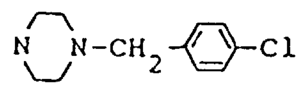

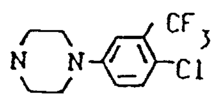

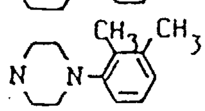

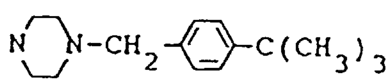

**18.** Composé selon la revendication 17 dans lequel $NR_1R_2$ représente

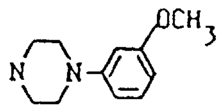

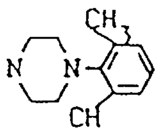

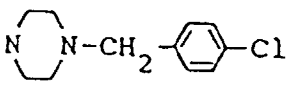

**19.** Composé de formule générale I

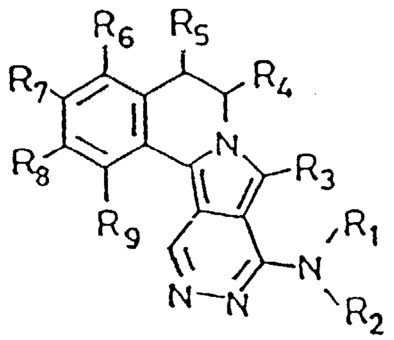

(I)

dans lequel $R_3$, $R_4$, $R_5$, $R_6$ et $R_9$ sont l'hydrogène, $R_7$ et $R_8$ sont alcoxy de 1 à 4 atomes de carbone ou $R_7$ et $R_8$ sont ensemble $-OCH_2O-$ ou $-OCH_2CH_2O-$ et le groupe $-NR_1R_2$ représente

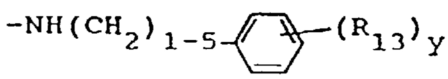

ou $-NH(CH_2)_{1\ \text{ou}\ 2}CH(C_6H_5)_2$, où $R_{13}$ est $CF_3$, $C(CH_3)_3$ ou $-OCH_2C_6H_5$ et y est 1 ou 2, ou ses sels physiologiquement acceptables avec des acides ou des complexants.

**20.** Composé selon la revendication 19 dans lequel $-NR_1R_2$ représente le groupe

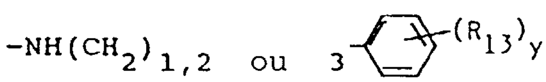

où $R_{13}$ et y sont définis comme dans la revendication 19.

**21.** Composé selon l'une des revendications 19 à 20 dans lequel $R_7$ et $R_8$ sont méthoxy.

**22.** Composé selon la revendication 19 dans lequel $R_7$ et $R_8$ sont méthoxy et $-NR_1R_2$ a l'une des significations suivantes :

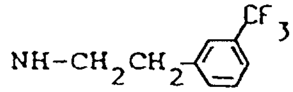

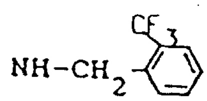

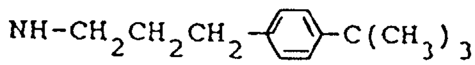

$NH-CH_2-CH_2-CH(C_6H_5)_2$

$NH-CH_2-CH(C_6H_5)_2$

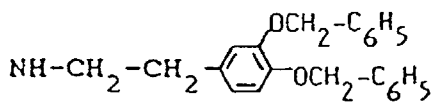

**23.** Composé de formule générale I

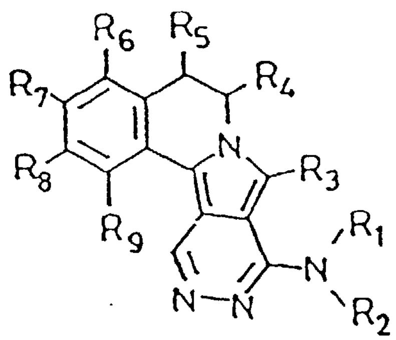

(I)

dans laquelle $R_3$, $R_4$, $R_5$, $R_6$ et $R_9$ sont l'hydrogène, $R_7$ et $R_8$ sont méthoxy et $-NR_1R_2$ a l'une des significations suivantes :

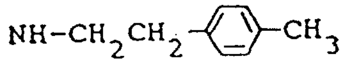

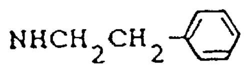

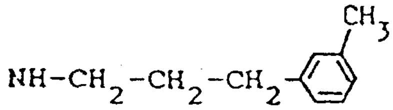

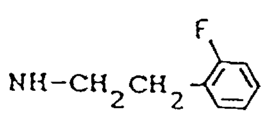

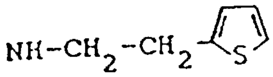

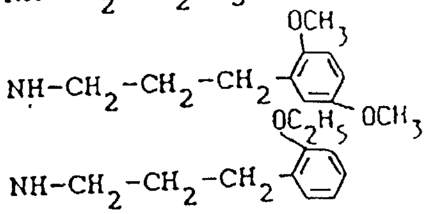

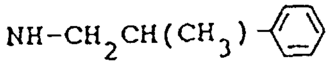

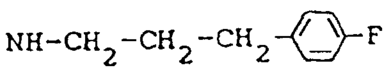

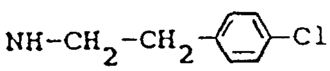

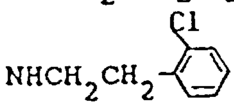

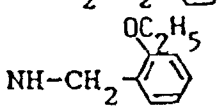

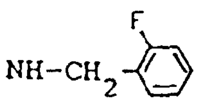

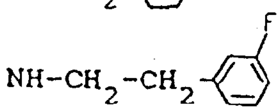

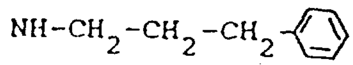

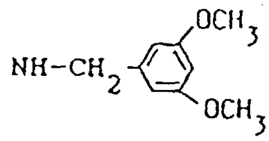

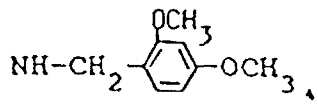

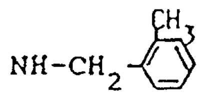

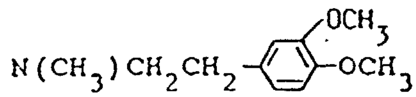

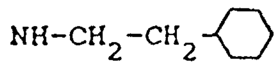

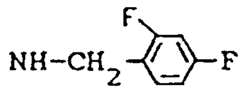

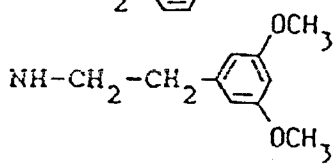

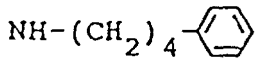

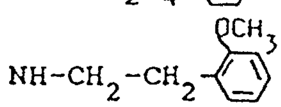

ou ses sels physiologiquement acceptables avec des acides ou des complexants.

**24.** Composé selon la revendication 23 dans lequel $-NR_1R_2$ a l'une des significations suivantes :

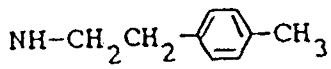

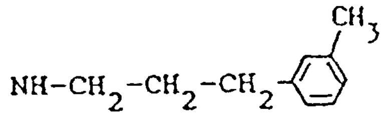

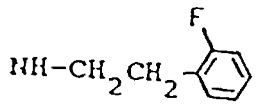

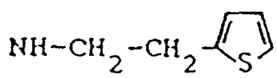

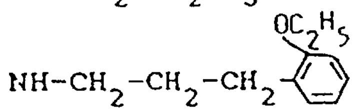

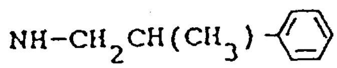

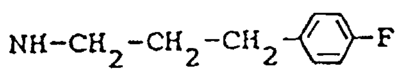

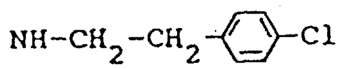

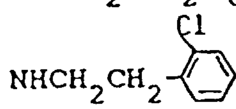

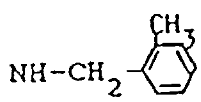

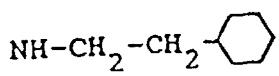

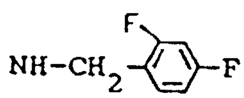

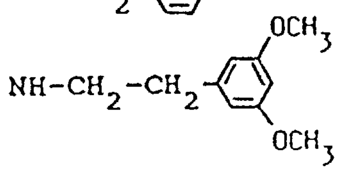

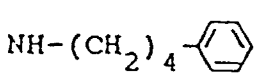 .

**25.** Procédé de préparation de composés de formule générale I selon l'une des revendications 13 à 24 ou de leurs sels physiologiquement acceptables avec des acides ou des complexants caractérisé en ce que l'on fait réagir un composé de formule générale II

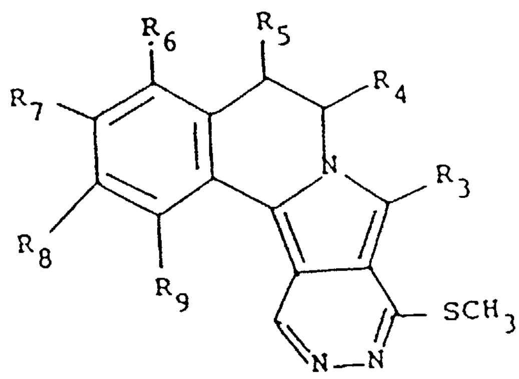

(II)

dans laquelle
les restes $R_3$, $R_4$, $R_5$, $R_6$, $R_7$, $R_8$ et $R_9$ ont les significations indiquées ci-dessus, avec un composé de formule générale

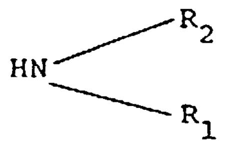

(III)

dans laquelle
$R_1$ et $R_2$ ont la signification indiquée ci-dessus, et en ce que l'on convertit éventuellement, de manière connue en soi, un produit final ainsi obtenu en un sel physiologiquement acceptable.

**26.** Préparations pharmaceutiques contenant comme principe actif un ou plusieurs composés selon l'une des revendications 13 à 24 en combinaison avec des adjuvants ou supports habituels.

**27.** Procédé de fabrication de préparations pharmaceutiques selon la revendication 26 caractérisé en ce que l'on convertit en formes d'utilisation pharmaceutiques habituelles des composés selon l'une des revendications 13 à 24 avec des adjuvants et/ou des supports galéniques habituels.